# EUROPEAN PATENT SPECIFICATION

(11) **EP 0 697 916 B1**
(45) Date of publication and mention of the grant of the patent: **05.06.2002**
(21) Application number: 95912699.6
(22) Date of filing: 03.03.1995
(51) Int. Cl.: B03C 1/01, B03C 1/32, G01N 33/553, G01N 33/543, G01N 35/00

(54) **METHOD AND APPARATUS FOR SEMI-AUTOMATED CELL SEPARATION**
VERFAHREN UND APPARAT ZUM SEMI-AUTOMATISIERTEN TRENNEN VON ZELLEN
PROCEDE ET APPAREIL POUR UNE SEPARATION SEMI-AUTOMATIQUE CE CELLULES

(30) Priority: 14.03.1994 US 212479; 14.03.1994 US 212616
(43) Date of publication of application: 28.02.1996
(73) Proprietor: Nexell Therapeutics Inc., Irvine, CA 92618-1605 (US)
(72) Inventor: MOUBAYED, Ahmad-Maher, Mission Viejo, CA 92691 (US); BURGESS, Julie, D., Long Beach, CA 90803 (US); LAW, Ping, Laguna Hills, CA 92653 (US); KULCINSKI, David, L., Santa Ana, CA 92701 (US); HARDWICK, Alan, R., Lake Forest, CA 92630 (US)
(74) Representative: Bassett, Richard Simon
(86) International application number: US9502642
(87) International publication number: WO9524969

(56) References cited:
- WO-A-90/04019
- WO-A-91/04059
- US-A- 4 710 472
- US-A- 4 861 553
- US-A- 4 910 148
- US-A- 5 336 760
- Bone Marrow Transplantation, Volume 3, issued 1988, KVALHEIM et al., "Immunomagnetic Removal of B-Lymphoma Cells from Human Bone Marrow: a Procedure for Clinical Use", pages 31-41, see figure 1, page 33.

## Description

### Background of the Invention

### Technical Field

The present invention is in the field of apparatus and method for magnetic cell separation. More particularly, magnetic separation of viable desired cells (target cells) from a heterogeneous cell mixture is accomplished by magnetic separation of desired cells from unwanted cells and other materials while the target cells are bound to paramagnetic beads, forming cell/bead complexes, followed by unbinding of the paramagnetic beads from the target cells, and removal of the target cells from the paramagnetic beads.

### Related Technology

In the field of cell separation, it is common to separate cells from plasma in blood or bone marrow, and also to separate, by centrifugation, white cells from red cells and platelets. The white cell population separated by standard centrifugation techniques is known as the "buffy coat" fraction. The buffy coat fraction excludes hemoglobin-containing eurythrocytic cells as well as platelets. However, the buffy coat fraction does contain a wide mix of hematopoietic mononuclear cells (MNC), including stem cells and progenitors of the erythrocyte, lymphocyte, monocyte, macrophage, granulocyte, and megakaryocyte lineages.

The various types of hematopoietic mononuclear cells are of nearly equal specific gravity, and thus they may not be separated from each other by centrifugation alone. However, different cell types have different sets of cell-surface markers such as specific proteins and glycoproteins on their surfaces. Thus, cell separation techniques may exploit selective binding to the desired cell type, also known as the "target cell".

For example, the hematopoietic stem cell is often the desired "target cell" because its progeny have the capacity to differentiate into all the different types of hematopoietic cells. In theory, it is possible to reconstitute a patient's hematopoietic system by infusing a concentrated suspension of stem cells. A stem cell marker known as "CD34 cell surface antigen" may be exploited to separate human hematopoietic stem cells from a mixture of mononuclear cells.

Once positive separation of stem cells becomes practical, new fields of therapy become possible. For instance, a patient's stem cells may be selected, induced to proliferate in an ex vivo culture, and then be returned to the patient after radiation or high dose chemotherapy treatments, which destroy the rapidly-dividing hematopoietic cells of the bone marrow. The selected stem cells may also be induced to differentiate ex vivo to produce more mature cells such as neutrophil and megakaryocyte precursors, which then may be returned to the patient in order to reduce bacterial infections and episodes of bleeding. A concentrated suspension of stem cells would also provide a natural host for gene therapy. For instance, a patient's selected stem cells could be transfected with a gene which, when expressed, could cure a genetic disease of the hematopoietic system such as sickle cell anemia or chronic granulomatous disease. Stem cells also could be transfected with genes which could cure genetic diseases outside of the hematopoietic system. For instance, certain forms of hemophilia could be cured if a small number of the patients' stem cells could be made to produce only a very small amount of certain clotting factors. Also, adjunct cancer therapies are envisioned whereby a patient's stem cells are transfected with a gene which provides resistance to chemotherapeutic agents. After these transfected stem cells repopulate the patient's bone marrow, the patient may be given high-dose chemotherapy for metastatic breast or colon cancer, for instance. The chemotherapeutic drugs will target rapidly dividing metastatic cancer cells, but hematopoietic cells of the bone marrow will be spared because they express a gene which disables the drugs.

The field of cell separation has been divided into two general categories: negative cell separation and positive cell separation. In negative cell separation, the cells that are bound in the device are deleterious cells such as tumor cells that are to be purged from a heterogeneous cell mixture such as blood or bone marrow, and which subsequently is returned to the patient. Thus, in negative separation, it is not critical to maintain the viability of the bound cells. Rather, in negative separation, it is critical to remove 100% of the tumor cells while maintaining the viability of the unbound cells. Throughout this application, it is to be understood that the term "bound cells", when used with respect to the use of paramagnetic microbeads, refers to cells which are attached to or are bound to such microbeads to form a cell/microbead complex. The cell/microbead complexes, for example, may include several cells bound to each microbead, or may include single-cell complexes in which only a single cell is bound to a particular microbead.

Positive cell separation presents special challenges in that it is critical to maintain the viability of the bound cells. One approach to positive cell separation involves the use of an avidin column to which biotinylated secondary antibodies are bound, as may be seen in the following publications: EP 260 280 B1; WO 92/07243; WO 91/16116; WO 91/16088; WO 93/08258.

The heterogeneous cell mixture is first incubated with primary antibodies against CD34, for instance, which specifically binds to stem cells. The cell mixture then flows through the column such that the secondary antibodies on the column bind to the primary antibodies, which in turn are bound to the desired cells. The undesired cells travel through the column with the elution fraction for subsequent disposal. United States patent 5,240,856 (Goffe, et al.), discloses the use of a magnetic stir bar to mix the cell suspension during column processing. The positively selected cells are then mechanically dislodged from the column. However, there are problems associated with this column-based system such as low cell viability, possibly due to mechanical damage; and of low cell purity and yield, possibly due to entrapping of the cells in the column.

Other current practices in the field for cell separation utilize matrix materials of, for example, hollow fibers, flat sheet membrane, or packed-bed bead or particle materials with physically adsorbed or covalently attached chemicals or antibodies for selective cell separation. These devices are designed to allow continuous whole blood or blood component inflow and return. Since these devices operate at normal blood flow rates under conditions in which the concentration of desired cells can be very low compared with other cell types, the separation process is often not efficient. Moreover, with these systems it is difficult to collect the selected cells in a viable state.

The development of paramagnetic beads offered the prospect of magnetic separation of target cells. Various methods to produce magnetic and paramagnetic particles are disclosed in the following United States patents: 4,672,040 (Josephson); 5,091,206 (Wong, et al); 4,177,253 (Davies, et al); 4,454,234 (Czerlinski); 4,582,622 (Ikeda, et al); 4,452,773 (Molday); 5,076,950 (Ullman); 4,554,088 (Whitehead); and 4,695,392 (Whitehead).

Various methods were devised to use magnetic particles for assays. See, for example, United States patents: 4,272,510 (Smith, et al); 4,777,145 (Luotola, et al); 5,158,871 (Rossomando); 4,628,037 (Chagnon); 4,751,053 (Dodin); 4,988,618 (Li, et al); 5,183,638 (Wakatake); 4,018,886 (Giaever); and 4,141,687 (Forrest).

Attempts were made to use magnetic particles for separation of biological components, including cells. The following is a list of United States patents known to the Applicants and believed to be directed to magnetic separators and methods: 4,855,045 (Reed); 4,664,796 (Graham, et al.); 4,190,524 (Watson); 4,738,773 (Müller-Ruchholtz); 4,941,969 (Schönert); 5,053,344 (Zhorowski); 5,200,084 (Liberti); 4,375,407 (Kronick); 5,076,914 (Garaschenko); 4,595,494 (Kukuck); 4,290,528 (Stekly); 4,921,597 (Lurie); 5,108,933 (Liberti, et al.); 4,219,411 (Yen); 3,970,518 (Giaever); and 4,230,685 (Senyei). All of these devices and methods have met with only very limited success due to problems with efficiency of cell separation and retention, and low viability of processed cells.

In attempts to remedy the problems with cell separation, other researchers devised alternate magnetic separator devices, as evidenced by the following United States patents, which proposed, for example, adjustable magnet positions (4,710,472; Saur, et al), magnetic gradients (4,904,391; Freeman), and magnets of opposite polarity (4,910,148; Sorensen, et al). These proposed devices did not solve the two main problems generally encountered in magnetic negative and positive cell separation, i.e., first, the need for very high target cell separation which desirably approaches 100% from a heterogeneous population containing a very low percentage of target cells; and second, the need for removal of nearly all the paramagnetic beads from the final cell product. Thus, these proposed conventional magnetic separator devices have not met with much success for either negative or positive cell separation.

The central problems for both negative and positive cell separation as described above were solved by utilizing two different magnets in series (Hardwick, et al., Artificial Organs 14:342-347, 1990; EP 0438520). In this invention, the first magnet has a relatively strong surface magnetic field strength combined with a broad "reach out" capacity such that its magnetic attractive force acts across the depth of the first container to magnetically attract and bind a high proportion of the paramagnetic particles in the first container. The second magnet in the series has a much stronger magnetic field strength at its surface than the first magnet, such that any paramagnetic beads which escape the first magnet are captured by the second magnet, and thus are removed from the final cell suspension product.

This invention by Hardwick, et al., solved the central problems for cell separation, in general, and for negative cell separation in particular. However, positive cell separation posed an additional challenge in that a greater yield of viable selected cells was desired. Positive cell separation was made practical by inventive changes in the first magnet and in the first container such that desired cells are not crushed as they are drawn on paramagnetic beads to the first magnet (Hardwick, et al., "Design of Large-Scale Separation Systems for Positive and Negative Immunomagnetic Selection of Cells Using Superparamagnetic Mircospheres", J. Hematotherapy 1:379-386, 1992; Hardwick, et al., Adv. in Bone Marrow Purging, Wiley-Liss, Inc., pages 583-589, 1992.) The preferred first magnet for positive cell separation retains certain of the characteristics of the first magnet for negative cell separation in that its "reach out" capacity is sufficiently broad to magnetically attract paramagnetic beads across the depth of the first container. However, the first magnet for positive cell separation has a much lower magnetic field strength at its surface than does the first magnet used for negative cell separation. Moreover, the first container for positive cell separation is non-collapsible. This combination of first magnet and non-collapsible first container permits a much greater yield of viable desired cells. In positive as well as negative cell separation, the second magnet has a much stronger magnetic field strength at its surface than the first magnet.

The system for positive cell separation described immediately above is manually operated, and may be considered somewhat labor intensive. As with all manual systems, the results obtained depend greatly upon the skill of the operator, and may not be as repeatable as is desired. There remains a need for a cell separation apparatus which is suitable for a clinical laboratory with limited available labor. Such an apparatus would preferably be semi-automated such that the operator would be free to attend to other duties during most of the cell separation procedure. Also, this semi-automated apparatus would improve the reliability and repeatability of the cell separation process by insuring that the operator performs the complex cell separation process correctly each time. Also, the repeatability of the process and quality of results would be improved by such a semi-automated apparatus because the performance of the many steps in the process would be less dependent upon operator skill level, and less affected by the level of the operator's attention to the process.

Accordingly, a primary object for this invention is to provide a semi-automated method of magnetic cell separation.

Another object for this invention is to provide a semi-automated method of cell separation which will prompt a human attendant at each step of the method which is to be performed by the human.

Still another object for the present invention is to provide such a method which includes use of a machine having a programmable capability for both semi-automating the machine to perform part of the process of cell separation, and for monitoring the cell separation process and instructing and verifying performance of a human attendant who performs some of the steps of the process.

Another objective for the present invention is to provide a method of semi-automated cell separation and a disposable apparatus set in which the cell solution and materials for cell separation are handled and contained during the process;

Still another objective is to provide a method using such a machine with a programmable capability, along with software for especially configuring the machine to semi-automate the cell separation process to be carried out by cooperation of the semi-automated machine and a human attendant. The machine is used to both monitor the cell separation process, and to provide prompts or instructions at each step of the process to the human attendant who follows these prompts or instructions without having to personally learn the many steps of the process or remember during a particular cell separation process which of the several steps have been performed and which step is next to be performed.

These and other objects and advantages of the present invention will be apparent from a reading of the following detailed description of a single exemplary preferred embodiment of the invention, taken in conjunction with the appended drawing Figures, in which the same reference numeral refers to the same feature throughout the drawing Figures, or to features which are analogous in structure or function.

### Brief Description of the Drawing Figures

Figure 1 provides a front quarter perspective view of a combined magnetic cell separation apparatus to be used in the present invention, which combined apparatus includes a semi-automatic machine, and a disposable apparatus set of processing chambers with permanently interconnecting tubing and associated valves and fittings;

Figure 2 provides a view of the disposable apparatus set to be used in the present invention for use with the machine of Figure 1, shown laid out as though on a table to better depict the structure and interconnection of the various parts of the disposable apparatus set;

Figure 3 is a diagrammatic representation of the mechanism, motors, control circuits, and operator input/output facilities of the machine seen in Figure 1;

Figure 4 provides a fragmentary cross sectional view taken generally at the plane 4-4 of Figure 1, and with operative parts of the machine shown in an alternative operational position;

Figure 5 is a diagrammatic fragmentary representation of a portion of the machine seen in Figure 1, and depicts in solid and phantom lines the oscillating rocking motion which is provided by a portion of the machine;

Figure 6 is a diagrammatic fragmentary representation of a portion of the machine seen in Figure 1, and depicts the machine portion selectively positioned for carrying out a step in the process of magnetic cell separation;

Figure 7 provides another diagrammatic representation of the mechanism of the machine seen in Figure 1;

Figure 8 is a fragmentary perspective view of the machine of Figure 1, with parts of the machine shown in alternative operative positions for clarity of illustration;

Figure 9 provides a top-level flow chart of the semi-automated process of the invention which is carried out with the machine and apparatus of Figures 1-8; and

Figures 10 - 17, provide successive sections of a high-level flow chart of the semi-automated process to be carried out by the machine of Figure 1 with a human attendant, and the disposable apparatus set of Figure 2, which flow chart is at a level below the top-level chart of Figure 9.

### Detailed Description of an Exemplary Preferred Embodiment of the Invention An Overview

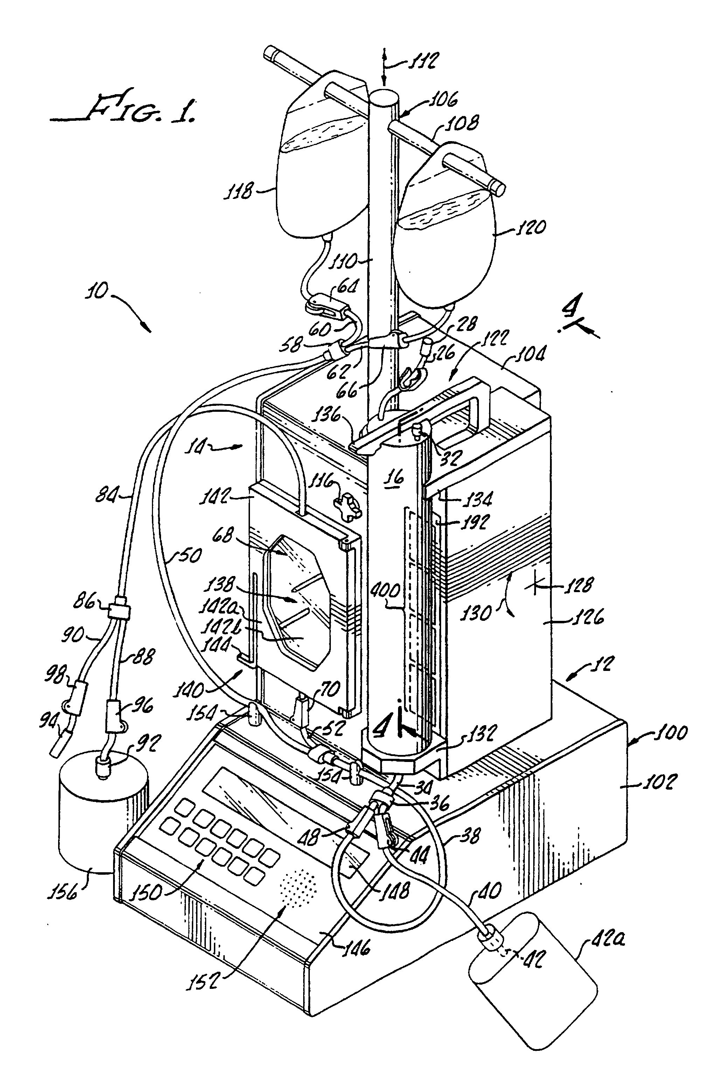

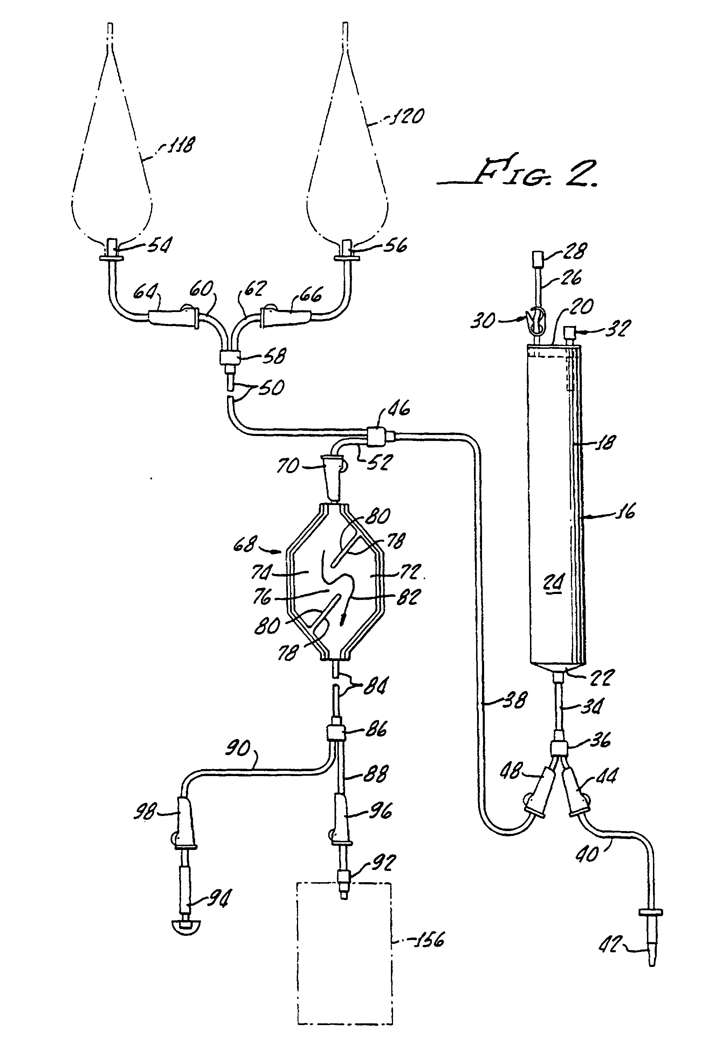

Viewing first Figures 1 and 2 in conjunction with one another, a combined apparatus for magnetic separation of cells is generally indicated with the numeral 10. This combined apparatus 10 includes a semi-automated machine 12, and a disposable and replaceable apparatus set 14. The disposable apparatus set 14, and the machine 12 are constructed and configured to be used together in cooperation to carry out a semi-automated magnetic separation of target cells, as will be further explained. Following a magnetic target cell separation process, the disposable apparatus set 14 is removed from the machine 12 and is discarded. Thereafter, another substantially identical disposable apparatus set 14 is installed on the machine 12 in preparation for a next subsequent magnetic cell separation.

### Disposable Apparatus Set 14

The disposable apparatus set 14 is shown in Figure 1 installed on the machine 12. However, in order to gain a better understanding of this disposable apparatus set 12, and of its use in the process of magnetic cell separation in conjunction with the machine 12, attention is directed now to Figure 2. The disposable apparatus set 14 includes a non-collapsible shape-retaining transparent cylindrical primary container 16. It is understood that the primary container of the invention may have an alternative geometrical form other than a cylinder, such as a generally rectangular volumetric form. This primary container 16 includes a transparent tubular body 18 having upper and lower end walls 20 and 22, respectively, which are sealingly joined permanently to the body 18 in order to define a primary processing chamber 24. Along the vertical length of the tubular body 18 are disposed three level marks, respectively indicated with the indicia "B", "A", and "L", from bottom to top of the chamber 24. These indicia will be used by a human attendant of the machine 12 to judge volumes of liquid combined with other materials (such as cells and paramagnetic beads) in the chamber 24 during the cell separation process, and especially during the adding of certain liquid materials to the contents of the chamber 24.

Permanently attached at the upper wall 20, and communicating with the chamber 24, is a vertically extending vent tube 26. This vent tube 26 extends to and carries a vent filter 28. On the vent tube 26 is carried a vent control clamp 30, which is effective to collapse and close the tube 26, as well as to allow this tube to open due to its own elasticity when the clamp 30 is released to allow venting of chamber 24. Also permanently attached on the upper wall 20, and communicating with the chamber 24, is an injection fitting 32, which includes a penetrable septum (not shown) to allow for injection of material into the chamber 24 through the septum via use of a hypodermic needle and syringe.

At the bottom wall 22, and communicating with the chamber 24, is permanently connected a short length of flexible tubing 34. This flexible tubing 34 extends to and is permanently joined to a y-connector 36. From the y-connector 36 extends a pair of permanently connected tubes 38 and 40. The tube 40 defines an outlet drain line for the disposable apparatus 14, and carries a permanently attached spike connector 42. On the drain line 40 is carried a roller clamp 44, which is effective to close communication from the chamber 24 to the drain outlet 42 by collapsing the tube 40, and to allow this tube to open also. The tube 38 extends to a permanently attached y-connector 46, and a roller clamp 48 controls communication through the tube 38. From the y-connector 46 extends two permanently attached tubes 50 and 52. The tube 50 communicates with a pair of inlet spike connectors 54, and 56, via a permanently connected y-connector 58, and a pair of tubes 60, and 62 connected permanently thereto. On the tubes 60 and 62 are carried respective roller clamps 64, and 66 to control communication through these tubes.

The tube 52 extends to a permanently attached secondary container 68. A respective roller clamp 70 controls communication through the tube 52. The secondary container 68 is formed as a flexible bag of octagonal shape as seen in the viewing aspect of Figure 2. This container has a pair of transparent closely-spaced and generally flat walls, indicated with the numerals 72, and 74, which are both generally one behind the other and in the plane of the Figure as seen in Figure 2. These confronting walls are joined at their octagonal periphery in confronting relation only slightly spaced apart in the direction perpendicular to the plane of the Figure to define a thin envelope-like shape for a secondary chamber 76. Within the container 68, the walls 72, 74 define respective ribs 78 which extend toward one another and are inter-bonded together to form a pair of angulated partitions, indicated with the numeral 80, within the chamber 76. Because of the partitions 80, the chamber 76 forms a serpentine flow path therethrough, which is indicated with the numeral 82, viewing Figure 2. It should be noted that the apparatus 14 is presented in Figure 2 as though it were laid out on a table, and that in the use of the machine 12 and apparatus 14, the flow path 82 extends upwardly through the chamber 76 so that fluid flow, and flow of material carried into chamber 76 by the flowing fluid, is against gravity. The importance of this flow against gravity in the chamber 76 will be explained further.

From the secondary container 68 a permanently attached tube 84 extends to a y-connector 86, which is also permanently attached to the tube 84 and to a pair of tubes 88, and 90. The tubes 88 and 90 respectively extend to a product outlet luer lock fitting 92, and to a product outlet ring cap fitting 94. Respective roller clamps 96 and 98 control the communication in tubes 88 and 90.

### Machine 12

Having observed the structure of the disposable apparatus set 14, attention may now be directed again to Figure 1, in which this disposable apparatus set is shown installed on the machine 12 preparatory to the conducting of a magnetic cell separation process with the combined apparatus 10. As is seen in Figure 1, the machine 12 includes a housing 100 having a base portion 102 and a vertically upwardly extending tower portion 104 upon the base 102. Telescopically received in the housing 100, and extending adjustably upwardly therefrom, is a T-shaped bag hanger 106. This bag hanger 106 is adjustable for vertical position of a cross bar portion 108 thereof by sliding a shaft portion 110 of the bag hanger 106 up or down relative to the tower portion 104, as is indicated by the arrow 112, and locking a selected position by use of a manual locking screw threadably carried by the housing 100 (not visible in the drawing Figures). The contoured external knob 116 of this locking screw is seen projecting from the front of the tower portion 104.

Upon the cross bar 108 are carried two bags 118, 120, one of prepared cell suspension and the other of buffering solution, respectively. The bags 118 and 120 are connected to the disposable apparatus set 14 at the inlet spike connectors 54 and 56. The primary container 16 is carried in upper and lower clamp features 122, 124, respectively, of a rocker assembly 126, which is journaled on one side of the tower 104. The rocker assembly 126 is pivotal about a pivot axis, generally indicated at 128, and as indicated by arrow 130, between the vertical position shown in Figure 1, and a limit position about 120 degrees clockwise from the vertical position, as will be further explained. The lower clamp feature 124 on the rocker assembly 126 takes the form of a cup-like bracket 132 extending outwardly from the rocker assembly 126 and having a slot for passage of the tube 34. The upper clamp feature 122 takes the form of an arcuate saddle member 134 disposed near the upper extent of the rocker assembly 126 for engaging the tubular body 18, and a spring-loaded cooperative latch arm 136 for retaining this first container 16 in engagement with the bracket 132 and saddle 134.

On the front surface of the tower 104 is carried a secondary magnet 138 and holder 140 for the secondary container 68. This holder 140 includes a door assembly 142, having a metallic frame portion 142a, defining a central opening within which is carried a transparent window portion 142b. The door 142 is provided with a pivotal door handle mechanism 144, both of which are visible in the depiction of Figure 1. The secondary container 68 is inserted into the holder 140, with the tube 52 extending into this holder via a lower notch (not seen in drawing Figure 1), and the tube 84 extending outwardly from this holder via an upper notch (also not visible in Figure 1). The tube 38 is sufficiently long in conjunction with the length of tube 34 to extend to the bottom of the first container 16, and to allow the movements of this chamber on rocker 126 through the full range of pivotal movement for this rocker assembly, as is described below.

At a front inclined panel 146 of the housing base 102 is disposed an operator annunciator screen 148, and input keypad 150. The annunciator screen 148 is preferably of a liquid crystal alpha-numeric type so that commands, signals, and directions to a human operator can be spelled out, and so that numerical values, such as count-down times, can be displayed for the operator. This front panel 146 also includes an audible alert-signal generator, such as a buzzer indicated with the arrow 152, for sending an auditory signal to the human attendant operator when a particular phase of the process is complete, or when a part of the process to be performed by a human attendant is required, for example. The human attendant will then silence the buzzer by an input to the key pad 150, and read the annunciated directions from the screen 148. It should be noted that the machine 12 can signal the operator for attendance either by an auditory signal, or simply with an annunciated signal or direction on screen 148. Above the inclined front panel 146, the housing base 102 carries a pair of upright tubing supports 154, which are used to organize and position the various tubes of the disposable apparatus set 14. Adjacent to the machine 12 is positioned a product container 156, which communicates with one of the product outlet fittings 92, 94. A waste container, depicted in Figure 1, and referenced with the numeral 42a, may preferably be connected to the waste drain connection 42.

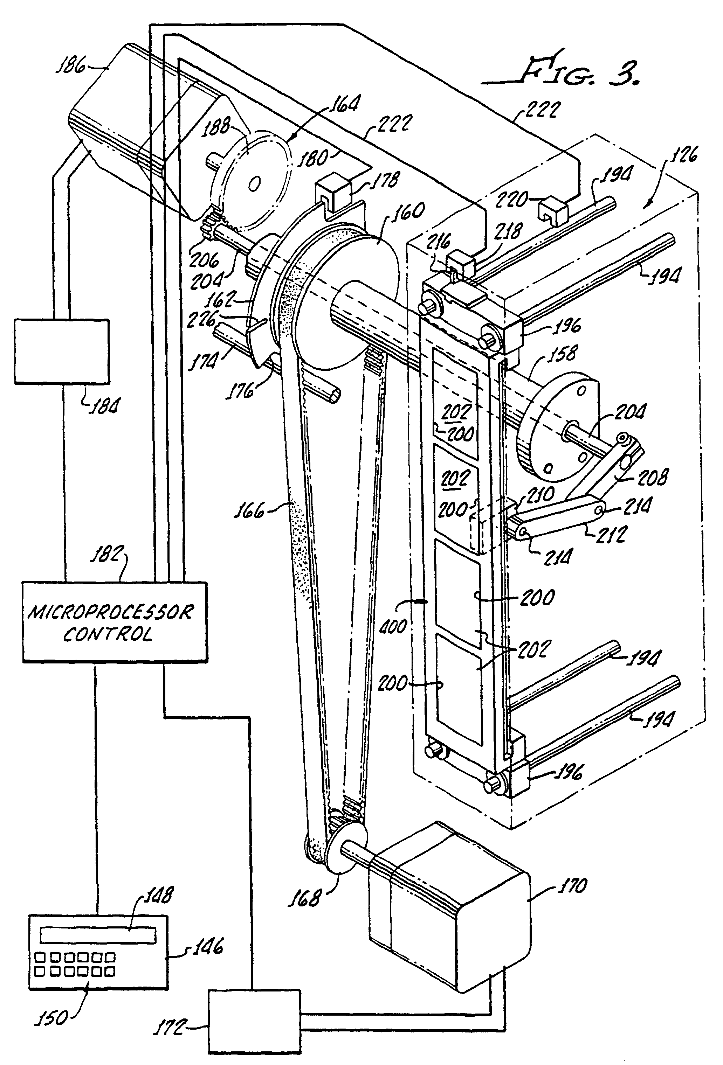

Turning now to Figure 3, a diagrammatic representation of the mechanization of the machine 12 is presented. As is seen in Figure 3, the tower portion 104 journals a tubular shaft member 158. The shaft member 158 carries the rocker assembly 126, and also drivingly carries a toothed pulley 160, an angular encoder and stop disk 162, and a mechanism, generally indicated with the numeral 164, for holding and positioning a primary magnet assembly (which is further described below). From the toothed pulley 160, a toothed timing belt 166 extends to a toothed pulley 168 which is drivingly carried on the output shaft of a motor 170. The pulleys 160, 168, in cooperation with the toothed belt 166 define a positive 3:1 gear ratio between the motor 170 and the rocker assembly 126. The motor 170, along with a motor controller 172 for this motor, is carried within the base portion 102 of the housing 100. The shaft member 158 defines the pivotal axis 128 for the rocker assembly 126, so that rotation of this shaft by the belt 166 and pulleys 160, 168 driven by motor 170 pivots the rocker assembly between its various positions, is effective to hold a selected position for the rocker assembly 126 because of magnetic cogging of the motor 170, and is effective also to angularly oscillate the rocker assembly 126 about the axis 128 so as to agitate or mix the contents of the primary container 16.

Within the tower portion 104, the housing 100 carries a stop rod 174, which is engagable by an edge surface 176 of the angular encoder and stop disk 162 when the rocker assembly 126 is rotated counter clockwise about the axis 128 (viewing Figures 1 and 3) substantially to the vertical position seen in these Figures. A sensor 178 is also carried by the housing 100 within tower portion 104, and is connected by appropriate conductors 180 to a microprocessor-based controller, indicated with the numeral 182. This microprocessor-based controller 182 also has connection with the motor controller 172, and with another similar motor controller 184. The motor controller 184 controls a gear-head stepper motor 186, which drivingly carries on an output shaft thereof a gear 188 forming a part of the mechanism 164. The microprocessor-based controller 182 also has a connection with the annunciator panel 146 and key pad 148, as is diagrammatically indicated viewing Figure 3.

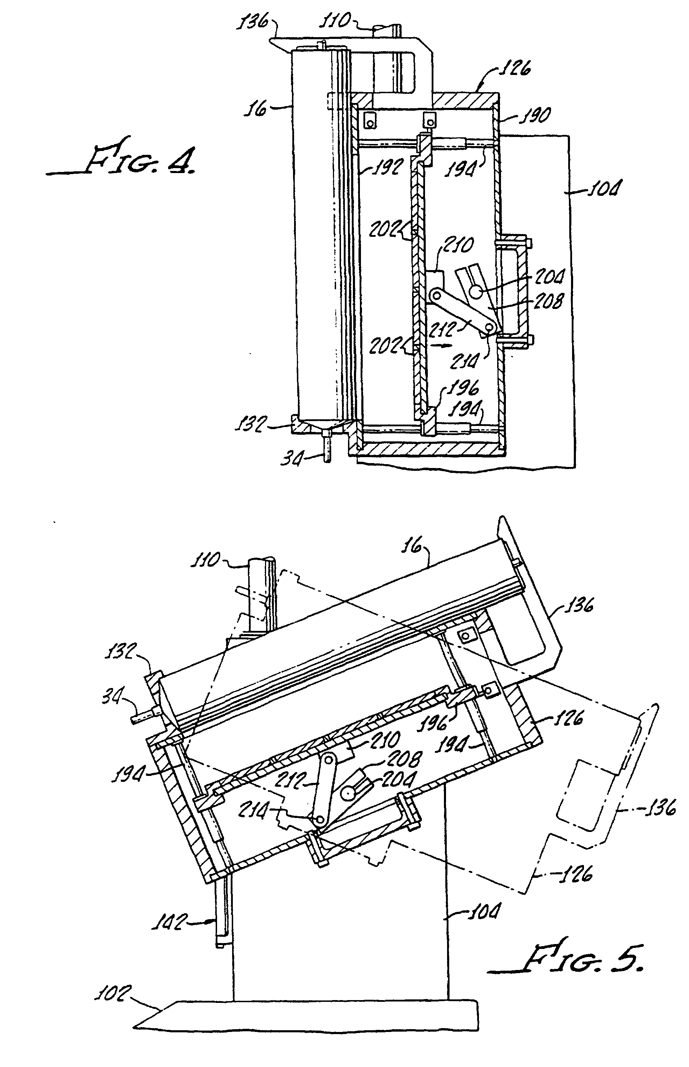

More closely considering the mechanism 164 for moving a primary magnet assembly 400, it is seen that the rocker assembly 126 consists of a hollow box-like housing 190, which is seen in phantom in Figure 3. The housing 190 defines a front opening 192, which is best seen in Figures 1 and 4. Within the hollow housing 190, four guide rods 194 extend from front to back of this housing outside of the boundaries of the opening 192. Slidably carried on the guide rods 194 is a primary magnet frame member 196. This frame member 196 on a protruding portion 198 thereof defines four magnet windows 200 within which respective primary permanent magnets 202 are captured to form a primary magnet assembly 400.

Suitably, each primary permanent magnet 202 is made of a high magnetism alloy of neodymium, iron, and boron, obtainable from the Crucible Magnetics Co., of Elizabethtown, Kentucky. In measuring the strength of a magnet, typically a gaussmeter is moved over the surface of the magnet, registering "peak flux density" also known as "local maxima" in gauss at various areas of the magnet. Each primary permanent magnet 202 has a magnetic field strength (local maxima) at its surface of preferably 2,000 to 3,000 gauss, most preferably 2,100 to 2,600 gauss. The primary permanent magnets 202 may be assembled with any combination of north or south poles facing inwards or outwards to form a primary magnet assembly 400. When the poles of each primary permanent magnet are arranged so that either all south poles face outwards, or so that all north poles face outwards, the primary magnet assembly 400 has a magnetic field strength (local maxima) of preferably about 3,000 gauss at a surface near either end of the primary magnet assembly 400, and a magnetic field strength (local maxima) of about 2,100 to about 2,600 gauss at a surface near the middle of the primary magnet assembly 400. When the primary permanent magnets 202 are arranged with alternating south and north poles facing outwards, the primary magnet assembly 400 has a magnetic field strength (local maxima) of about 4,000 gauss at a surface near an interface between north and south poles. It is within the scope of this invention to arrange the primary permanent magnets 202 with any permutation of north and south poles facing inwards or outwards to provide a primary magnet assembly 400 having magnetic field characteristics advantageous for different types of magnetic cell selection.

In a first or advanced position of the primary magnet frame member 196, the portion 198 thereof extends through the opening 192, so that the primary permanent magnets 202 are substantially flush against the tubular body member 18 of the primary container 16 of disposable apparatus set 14. This first position for the primary magnet assembly 400 is seen in Figures 1, 3, and 6.

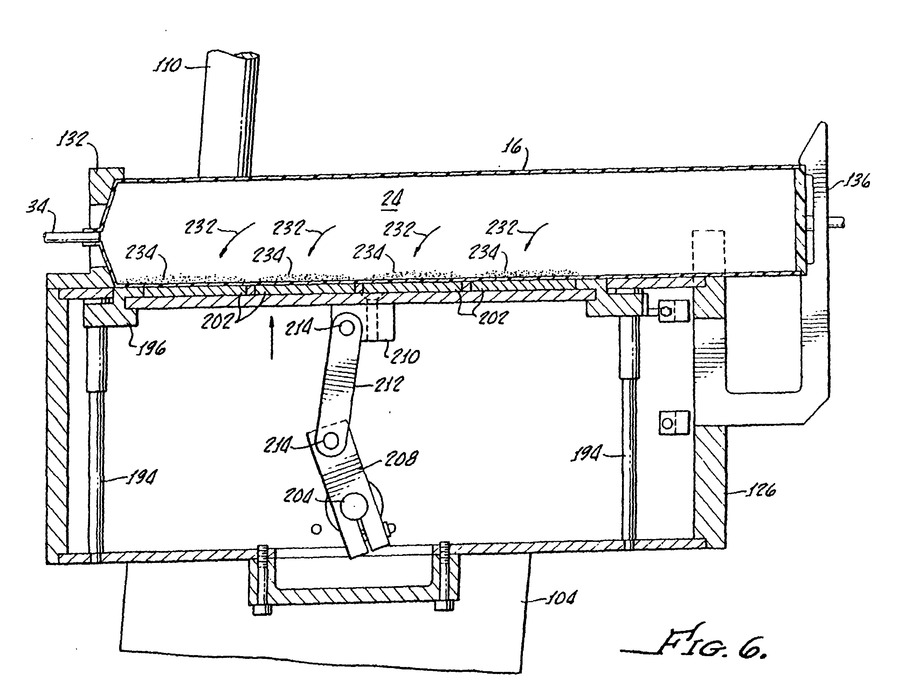

In order to move the primary magnet frame member 196, and the primary magnets 202 carried thereby, from the first advanced position illustrated in Figure 3, to a second or retracted position, seen in Figures 4 and 5, the mechanism 164 includes a shaft 204 journeled within the tubular shaft member 158. This shaft 204 carries a gear 206 meshing with the gear 188 of motor 186, and also carries an actuator arm 208 disposed within the rocker housing 190 behind the frame member 196. Between the distal end of the actuator arm 208 and a bracket 210 attached to the frame member 196 extends a link member 212. This link member 212 is pivotally connected to each of the actuator arm 208 and bracket 210 via associated pivot pins, generally indicated with the numeral 214. Consequently, when the shaft 204 is rotated counter clockwise, viewing Figure 3, by motor 186, and meshing gears 188, and 206, the actuator arm 208 pulls the frame member 196 and primary permanent magnets 202 away from the primary container 16 to the position indicated in Figure 4. Of course, opposite pivotal movement of the shaft 204 and actuator arm 208 advances the primary permanent magnets 202 to the position seen in Figures 1 and 3.

As will be further explained, the motor 186, and mechanism 164 are pivotal with the tubular shaft 158 and rocker member housing 190 so that pivotal movement of the rocker assembly 126 has no effect upon and is independent of the position of the magnet frame 196 and primary permanent magnets 202 relative to the primary container 16. Also seen in Figure 3 is a flag member 216 which is carried by and extends upwardly from the frame member 196. This flag member 216 is sensed by one or the other of two sensors 218, 220 in the respective advanced and retracted positions of the frame member 196. Each of the sensors 218, 220 has connection with the microprocessor-based controller 182 via appropriate conductors 222. Accordingly, the controller 182 will operate the motor 186 in the appropriate direction to move the primary permanent magnets 202 between their advanced and retracted positions. The primary permanent magnets 202 only occupy a position between their advanced and retracted positions while they are moving in traversing between these two positions. The primary permanent magnets 202 are not stopped at positions other than their advanced position or retracted position.

### Method of use of Machine 12 and Disposable Apparatus Set 14

Having now gained a general understanding of the machine 12 and disposable apparatus set 14, which together make up the combined apparatus 10, attention may now be directed both to additional structural features of the machine 12, which will be described in conjunction with a description of how the machine 12 and apparatus set 14 are used together in carrying out a process of magnetic cell separation.

Viewing Figures 1 and 4, it is seen that with the rocker assembly 126 in its vertical position, and with the primary magnets 202 retracted, a first phase of the magnetic cell separation process may be started by adding into the primary chamber 24 a small amount of the buffer solution from bag 120 and the cell mixture from bag 118. After emptying bag 118 into the primary chamber 24, the human operator may, if desired, flow a small amount of the buffer solution from bag 120 into bag 118 and then into the chamber 24 to rinse remaining cells from the bag 118 into the chamber 24. Buffer solution is then added to chamber 24 to bring the liquid level therein to one of the specified level lines. Into the chamber 24 via the injection port 32 is then placed a calculated quantity of paramagnetic beads. All of the clamps controlling liquid flow into and out of the primary chamber 24 are then closed.

When the operator indicates to machine 12 by an appropriate input at key pad 150 that this first preparatory activity is completed, the microprocessor based controller 182 moves the rocker assembly 126 from the vertical position shown in Figures 1 and 4 through a first agitation position (which is illustrated by the solid line position seen in Figure 5), on through the horizontal position, and beyond this horizontal position to a second agitation position (seen in phantom lines in Figure 5). This first agitation position of the rocker assembly 126 is preferably about 22.5 degrees short of a horizontal position for the container 16, while the second agitation position is 22.5 degrees beyond the horizontal with respect to the vertical position for primary chamber 16 which is seen in Figure 1. The way by which the machine 12 locates these angular first and second agitation positions is described below. However, the operator can pre-program the controller 182 to employ another first agitation position for the rocker assembly 126 if desired. The machine 12 is configured to allow a first agitation position which is any where in the range from just short of the horizontal to 30 degrees from horizontal. From the first agitation position, the machine 12 pivots the rocker assembly 126 clockwise viewing Figure 5, through the horizontal, and to an equivalently-angulated second agitation position, which is shown in phantom lines in Figure 5.

During a selected time interval, the machine 12 continuously oscillates the primary container 16 back and forth between the first and second agitation positions shown in Figure 5 at a rate which is also variable and may be preprogrammed. Of course, each oscillation of the primary container 16 causes the liquid contents of this container to flow or surge back and forth from end to end of the chamber 24, thoroughly agitating and mixing the contents of this primary chamber. This initial agitation interval results in the appropriately coated paramagnetic beads binding to all or substantially all of the target cells in the chamber 24. This rocking of the primary container 16 and agitation of the contents of chamber 24 is achieved by repeated reversals of the direction of rotation of motor 170. During this time, the primary magnets 202 are retracted into the housing 190 so that the magnetic field from these magnets does not affect the paramagnetic beads.

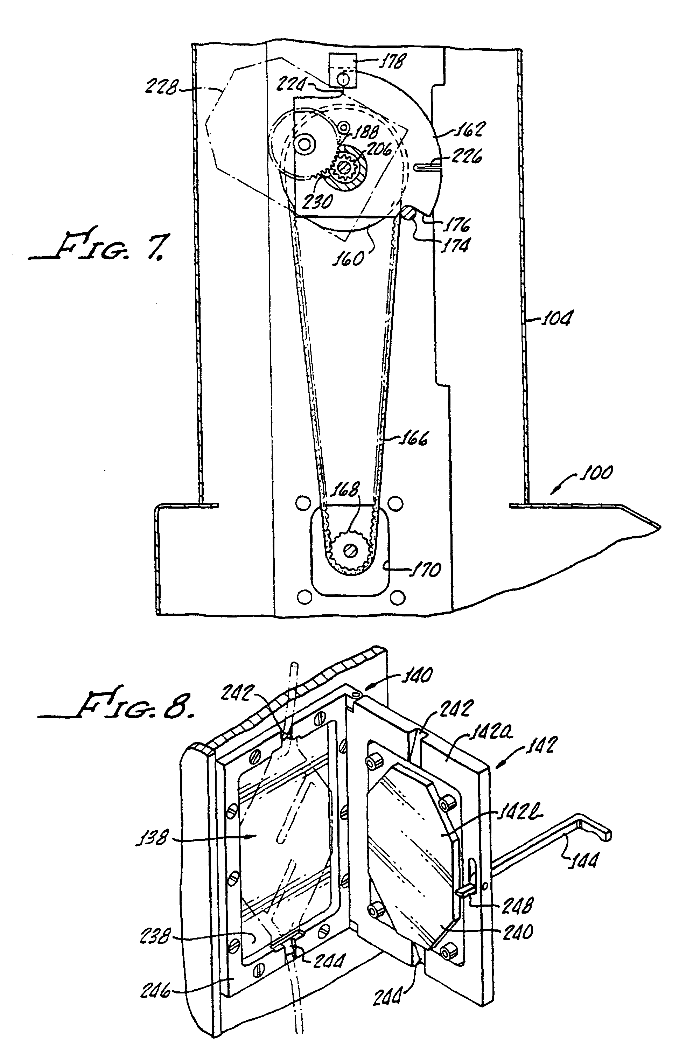

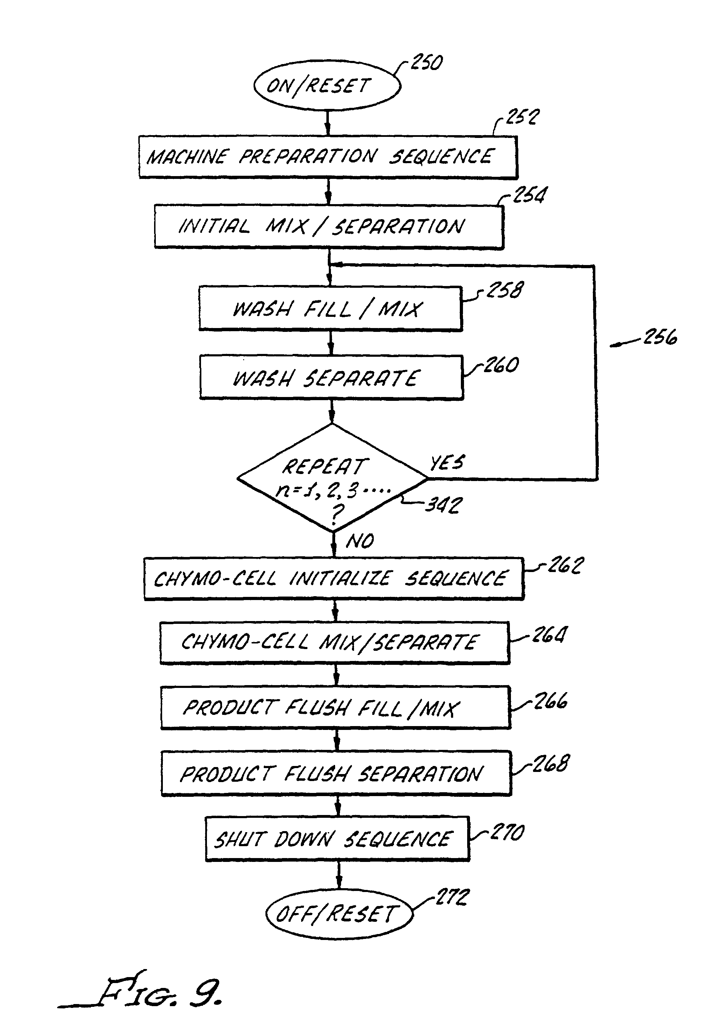

Attention now to Figure 7 will assist the reader in understanding how the controller 182 is enabled to control the rocker assembly 126 to locate the vertical position for this assembly, to locate the first and second agitation position, and to position this rocker assembly in a separation position which will be described. Figure 7 presents another somewhat diagrammatic representation of the mechanization of the machine 12, but is taken from the opposite side from the view of Figure 3, with the rocker assembly 126 also in the vertical position. Viewing Figure 7, it is seen again that in the vertical position for the rocker assembly, the edge surface 176 of disk 162 confronts the stop rod 174, but preferably does not actually contact this stop rod. Also, at the vertical position, the sensor 178 is just clear of an edge 224 of the encoder/stop disk 162. When the motor 170 pivots the rocker assembly 126 counter clockwise viewing Figure 7 (clockwise viewing Figures 1 and 3) to the horizontal position, a slit 226 in the encoder/stop disk 162 aligns with and is sensed by the sensor 178. From this horizontal position, the controller 182 pivots the rocker assembly back toward but just short of contact of the edge 176 with rod 174. This is the vertical position for the rocker assembly 126.

The motor 170 is preferably a stepper type of motor with a full-step rotation of 1.8 degrees of shaft rotation, and a half-step capability (i.e., the motor 170 can be commanded by controller 182 to rotate its output shaft in full-step units of 1.8 degrees of shaft rotation, or half steps of 0.9 degrees may also be commanded). In view of the 3:1 gear ratio between the motor 170 and shaft 158 which carries the rocker assembly 126, the controller 182 is programmed with the information that one step of the motor 170 equals 0.6 degrees of pivotal motion for the rocker assembly 126 (half-steps being equal to 0.3 degrees of motion). The controller 182 in an initial calibration phase of operation of the machine 12 will pivot the rocker assembly 126 from the horizontal position back toward contact between rod 174 and edge 176, stopping the rocker assembly just short of this contact. Thus, no matter what position the rocker assembly 126 happens to be in when the machine 12 is turned on and a calibration phase is conducted, the controller will thereafter have a reference for the horizontal position by reference to the slit 226, and can locate other positions for the rocker assembly 126 by counting steps and half steps for motor 170.

During rocking of the rocker assembly 126, the controller 182 counts the number of steps for motor 170 away from and back to the horizontal position, and this reference position is updated each time the slit 226 aligns with the sensor 178. The total angle through which the rocker assembly 126 is tilted during rocking is variable by the controller 126. As may be further explained, if the operator for the machine 12 commands that the rocker assembly be rocked through 50 degrees, for example, in order to agitate the contents of the container 16, then the controller 182 divides 50 by 2, and divides the result (25) by the 0.6 degree value for a full motor step. The closest integer value for the result (42), is the number of steps in each direction from the horizontal reference position (referenced to slit 226) which the controller 182 will command for the motor 170.

The preferred position of rocker assembly 126 for an initial phase of magnetic capture separation is about 5 degrees from the horizontal, and is also determined by reference to the horizontal reference position. The 0.6 degree per motor step value is divided into the preferred angular position for the rocker assembly to obtain a closest-integer value for the number of steps for the motor 170 to move the rocker assembly 126 from the horizontal position to the first magnetic capture position seen in Figure 6.

Figure 7 also shows that the mechanism 164 includes a motor mount housing 228 carried on the tubular shaft 158 for pivotal movement therewith. The tubular shaft 158 defines a window 230, at which the gear 206 is exposed. The gear 188 drivingly carried by the motor 186 meshes with the gear 206, and the motor 186 is secured to the housing 228 for pivotal movement with shaft 158. This motor mount housing 228 will preferably allow an angular movement for the rocker assembly 126 of about 30 degrees beyond the horizontal position before the motor mount housing 228 strikes the stop rod 174. Accordingly, the rocker assembly 126 may be rocked alternately in each opposite direction from the horizontal position about thirty degrees for symmetrical rocking motion, if desired. This larger 30 degree angle of rocking motion would require that the motor 170 make 50 steps between horizontal reference position and each extreme of tilting motion for the rocker assembly 126. Of course, a smaller angle of rocking for the rocker assembly 126 may be selected by appropriate programming of the controller 182.

Preferably, the slit 226 is positioned 30 degrees from the edge 176 of the encoder/stop disk 162. In the event that the operator of the machine 12 selects a rocking angle for the rocker assembly 126 which is 30 degrees, or close to this value, then the edge 176 will align with the sensor 178 when the rocker assembly 126 reaches its second agitation position of 30 degrees beyond the horizontal to provide a warning to the controller 182 that the motor housing 228 is close to the stop rod 174. The controller 182 will continuously update the position of the rocker assembly 126 with respect to the horizontal, and also receives a warning from the sensor 178 when the edge 176 reaches this sensor so that a collision between the motor housing 228 and the stop rod 174 does not occur during rocking agitation of the primary container 16.

Upon completion of the selected agitation interval, the controller 182 brings the rocker assembly 126 to a stop at an initial separation position for magnetic capture of the paramagnetic beads (and bound target cells) in the chamber 24. This initial separation position, or initial position for magnetic capture, is preferably about 5 degrees counter clockwise from the horizontal, viewing Figure 6. The primary magnets 202 are then advanced by operation of the motor 186 and mechanism 164, under the control of the controller 182. This separation position is held for a time interval which is preferably about 30 second, although another time interval of initial magnetic capture may be selected.

Immediately thereafter, the rocker assembly 126 is moved by controller 182 to a secondary separation position with the primary magnets 202 remaining in the advanced position seen in Figure 6. This secondary separation position is substantially vertical, as is seen in Figure 1. The applicants have found this two-phase separation process, which uses an initial inclined separation position for magnetic capture of the target cells and bound paramagnetic beads from the liquid in chamber 24, advantageously spreads out the liquid contents of the chamber 24 along the length of the primary magnet assembly 400 during the first phase of separation. As is indicated in Figure 6, the bound target cells tend to move as indicated by the arrows 232 into clumps on the wall of container 16, which clumps are indicated with the lead line and numeral 234. It will be noted that unbound paramagnetic beads and some non-target cells are also captured in the clumps 234. At this point, after the container 16 has been moved to its second separation position, and a second separation interval of time has passed, the operator is signaled by the machine 12 to vent and drain the chamber 24. Most of the non-target cells, and the liquid from bag 118 which carried these cells, is drained from the chamber 24 into a waste container.

The operator then begins the first wash of what may be a series of similar washing activities for the bound target cells from the clumps 234. That is, the chamber 24 is partially filled with buffer solution and is re-closed. The operator signals to machine 12 via key pad 150 that the primary processing container 16 is ready for further processing. Then the controller 182 retracts the magnets 202 to release the bound cells into the fresh buffer solution in chamber 24. The machine 12 agitates container 16 between the first and second agitation positions for a pre-selected time interval, and then magnetically separates the bound target cells into fresh clumps on the wall of container 16 as explained above.

These fresh clumps are like the clumps 234, but are of greater purity with respect to exclusion of non-target cells and other materials in the clumps. Again, the machine 12 completes the magnetic cell separation by capturing the bound target cells into clumps on the wall of container 16, moves the rocker assembly 126 to the vertical position for a secondary separation interval, and signals the operator to attend to draining the chamber 24. If another wash and separation cycle is to follow, the operator is instructed by an indication on the annunciator screen 148 to replenish the chamber 24 with fresh buffer solution. Preferably, the target cells are subjected to three wash/separation cycles so that at the completion of these wash/separation cycles the chamber 24 contains substantially only target cells bound on paramagnetic beads, and unbound paramagnetic beads.

When the wash/separation cycles are completed, the operator is instructed to refill the primary chamber 24 with buffer solution, and to inject via port 32 a releasing material such as the enzyme chymopapain. Chymopapain releases the target cells from the paramagnetic beads by enzymatic digestion of the proteins which mediate the binding of cells to beads. Alternative means of release are within the scope of this invention. For instance, other releasing materials could include small peptides, proteins, or other molecules which compete for binding at the antibody/antigen binding sites which mediate the binding of cells to beads. As another example for releasing means, the binding of cells to beads might be mediated by biotin/avidin binding, by biotin/anti-biotin binding, or by biotin analog/anti-biotin binding. In this latter example, the releasing material would be biotin.

The chamber 24 is again closed, and the machine 12 agitates the contents of the container 16 to incubate the bound target cells with the enzyme or other releasing material and to release all or substantially all of the target cells from the paramagnetic beads. At the completion of this agitation, the primary magnet assembly 400 is advanced and the two-phase magnetic capture and separation procedure is repeated. However, this separation step captures the paramagnetic beads on the wall of the container 16, while the unbound target cells remain free in the buffer solution. The operator is again signaled to perform the next step of the process.

The operator is instructed by the annunciator panel of machine 12 to drain the primary chamber 24 into a product-receiving container 156, viewing Figure 1, via the secondary chamber 76. The secondary magnet 138 has a magnetic field strength at its surface of at least about 7,000 gauss, and thus firmly traps in the secondary chamber 76 any paramagnetic beads which escape from the primary chamber 24. Also, the flow through chamber 76 is against gravity, which also assists in insuring that no beads pass through the chamber 76. Consequently, the product delivered to container 156 is rich in target cells and is substantially devoid of non-target cells and of paramagnetic beads.

Again, the operator will be instructed by annunciator panel 148 to partially refill the primary chamber 24 with buffer solution, and the machine 12 will conduct another agitation period during which target cells caught in the clumps of paramagnetic beads will be released into the buffer solution. Following this agitation interval, the magnets 202 will again be advanced, and a separation interval in the separation position of Figure 6 will capture the paramagnetic beads. The operator will again be summoned to drain the chamber 24 into the product-receiving container 156 via the secondary chamber 76. Again the paramagnetic beads will be trapped on the wall of container 16 or in the secondary container 68 while the target cells are delivered to container 156 so that the product yield of target cells is increased. Preferably, this washing of the paramagnetic beads in the primary chamber 24 to free additional target cells, which are subsequently added to the collection in container 156 is conducted at least once, but may be repeated several times if desired.

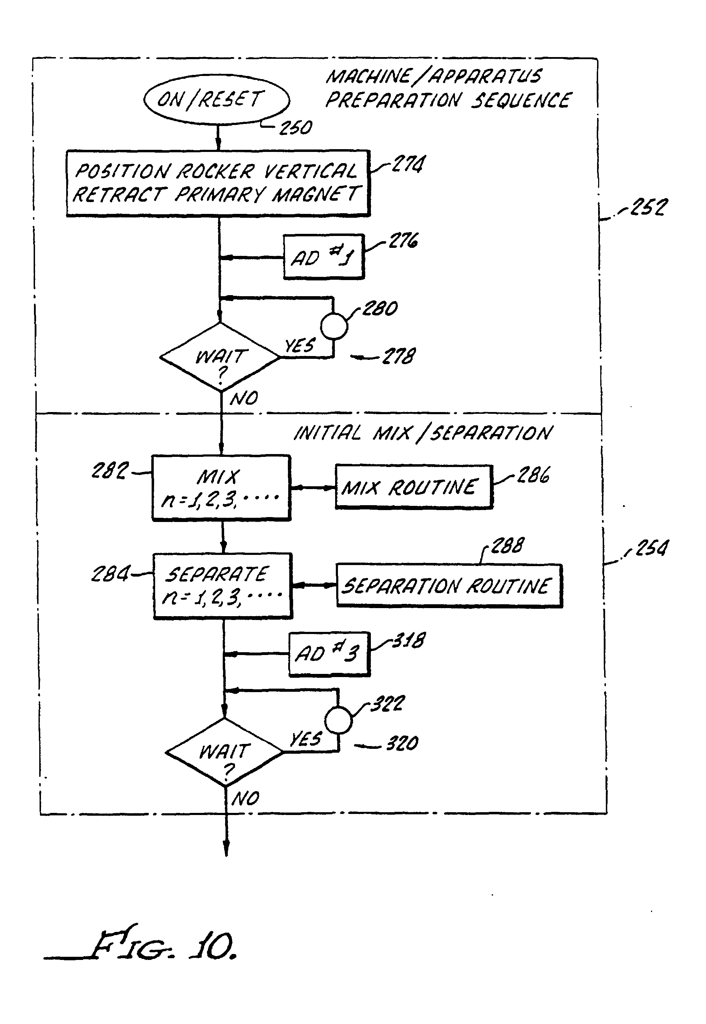

Attention now to Figure 8 will show that the secondary magnet 138 and holder 140 for the secondary container 68 cooperatively define a recess 238 for receiving the secondary container 68. The secondary magnet 138 defines a planar back wall for this recess 238, while the window portion 142b of the door assembly 142 defines a protruding hexagonal portion 240 extending into this recess 238 toward but short of the back wall defined by the secondary magnet 138. This portion 240 in cooperation with the back wall of the recess 238 defined by the secondary magnet 138 ensures that the secondary container 76 maintains a flat and thin configuration in close proximity to the secondary magnet 138 to ensure complete capture of paramagnetic beads passing into the secondary chamber 76.

The notches 242, 244 which were mentioned earlier as allowing passage of the tubes 52 and 84 into the holder 140 are seen to be defined in part by the frame portion 142a of the door 142, and in part by a rim 246 of the holder 140. The notches 242, 244 open across the rim 246 of the holder 140 into the recess 238. On the door 142 the door handle 144 includes an inwardly extending cam portion 248, which engages the underlying portion of the rim 246 when the outer part of the handle 144 is lifted to lever open the door assembly 142 in opposition to the strong magnetic field from the secondary magnet 138. Because a part of the door assembly 142 is made of magnetic metallic material, no other latching of the door is required. Further, the notches 242, 244 defined in the frame portion of the door assembly 142 can be used to receive the tubing sections 52 and 84 adjacent to the secondary container, holding this container to the protrusion 240 while the door 142 is closed.

Also, the levering action provided by the handle 144 and cam portion 248 is of assistance in moving the door frame 142a far enough away from the strong magnetic field of the secondary magnet 138 to allow the door 142 to be easily opened by hand. This configuration of the door 142 and door handle 144 with cam portion 248 also has an important advantage when installing the secondary container 62 into the recess 238. As mentioned above, the secondary container 62 is made essentially like a flat envelope or bag of transparent plastic sheet material. When this container is placed into the recess 238 and the door 142 is closed, there is an increasingly strong pull on the door by the secondary magnet 138. However, the door 142 should not be allowed to slam shut under this magnetic pull. Accordingly, the cam action of the handle 144 allows the operator to slowly close the door 142 while insuring that the container 62 is properly positioned in recess 238, and is not pinched.

### An overview of the Semi-Automated Cell Separation Process

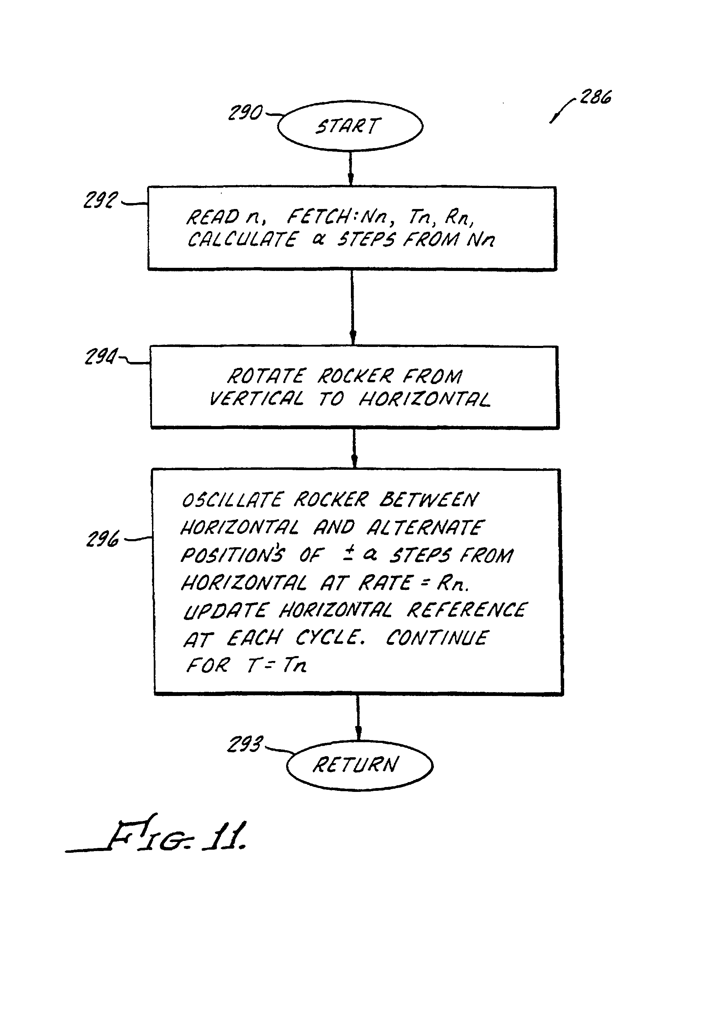

Viewing now Figure 9, a top-level flow chart is set out for the semi-automated cell separation process to be performed by the machine 12 of Figure 1 with the disposable apparatus set of Figure 2, and with the attendance of a human operator. As is seen viewing Figure 9, at the beginning of the process, the human attendant turns the machine 12, "on", as is indicated with the reference numeral 250. As is also indicated at 250, the human attendant's activity which starts a cell separation process cycle for the machine 12 may also be a "reset" command entered at the key pad 150 at the end of a previous cell separation cycle.

At this point in the process, as is generally indicated with the numeral 252, the machine 12 will prompt the operator to request positional calibration of the machine 12, which request when entered at the key pad 150, results in the machine 12 pivotally cycling the rocker assembly 126 from what ever position it happens to occupy when the machine is turned on, or from the vertical position seen in Figure 1, to its horizontal position (indicated by passage of slit 226 into alignment with sensor 178), retraction of the primary magnet assembly 400, and pivotal movement of the rocker assembly 126 back to its vertical position seen in Figure 1, as described above. At this time, the machine 12 also waits while a series of additional prompts are displayed to the operator. These prompts successively instruct the operator to perform: preparation of and installation of the disposable set 14 on the machine 12, priming of this set with buffer solution from the bag 120, addition of the cell mixture from bag 118 into the primary chamber 24, and addition of the paramagnetic beads via the port 32.

At this time, the operator may also be prompted to add a blocking agent via the injection port 32 into the primary processing chamber 24. The blocking agent may be a concentrate of pooled human immunoglobulins such as Gammagard® (Baxter Hyland) or another type of protein concentrate such as human serum albumin (Baxter Hyland). The addition of a blocking agent at this time assists in preventing the target cells and other cells in the cell mixture from adhering to one another, and prevents non-specific binding of non-target cells to antibodies and beads. The yield of viable target cells from the presently used positive immuno-magnetic cell separation process is increased by the addition of this blocking agent. At the completion of each activity, the operator is instructed to indicate to the machine 12 by way of the key pad 150 that the indicated step has been performed. The machine 12 then displays the next successive prompt to the operator, or begins its own part of the magnetic cell separation process when the human operator's activities in preparation to this activity have been completed, which ever is appropriate and according to the programming of the controller 182.

Briefly, the steps of the magnetic cell separation process to be conducted by the machine 12 include an initial mixing to bind the target cells with the paramagnetic beads, and an initial magnetic capturing of the bound target cells. The machine 12 will then prompt the operator to drain the buffering solution from the chamber 24, which also carries out of this chamber a large part of the non-target cells and other materials which initially entered chamber 24 from the bag 118 (this part of the process is generally indicated at 254 on Figure 9). This buffering material, as well as other materials to be drained to waste, are drained to a waste container, indicated with the numeral 42a on Figure 1.

Next, the machine 12 and the operator will cooperatively perform one or a series of washings of the bound target cells to successively remove substantially all of the non-target cells from the primary processing chamber 24. These washings each include the addition of fresh buffer solution to the primary processing chamber 24, the release of the bound target cell/bead complexes (as described above, and which are hereafter referred to as "bound cells"), as well as the release of non-target cells captured in the aggregations 234 of magnetically captured target cells into this fresh buffer solution, a short period of mixing, and the magnetic recapturing of the bound target cells followed by another draining of the buffering solution and non-target cells from the primary processing chamber 24 into container 42a. This washing, or series of washings, is generally indicated with the numeral 256 on Figure 9, and may be repeated a variable number of times as is preselected for the microprocessor-based controller 182 of the machine 12. More particularly, each washing cycle includes generally a fill and mix activity, indicated with the numeral 258, and a separation activity, which is generally indicated with the numeral 260 on Figure 9.

Once the bound target cells are adequately washed to remove substantially all non-target cells from the primary processing chamber 24, the machine 12 enters a releasing material initialize sequence (indicated at 262 on Figure 9) during which the machine 12 provides prompts to the operator to: again add fresh buffer solution to the chamber 24; and to add the releasing material such as the enzyme chymopapain. This releasing material will separate the target cells from the paramagnetic beads. This stage of the process is generally indicated with the numeral 262 on Figure 9.

The machine 12 will again release the magnetically captured bound target cells into the buffer solution, agitate these cells with the buffer solution with added releasing material to separate the target cells and paramagnetic beads, and again magnetically captures the paramagnetic beads (indicated generally at 264 on Figure 9). Significantly, the target cells, which are no longer bound to the paramagnetic beads, stay suspended in the buffer solution.

Again, the operator will be prompted by the machine 12 to drain the buffer solution from chamber 24. However, this time the operator will be instructed to drain the buffer solution and target cells from the primary chamber 24 into a product receptacle 156 seen in Figure 1, via the secondary processing chamber 76. As was pointed out above, this second processing chamber is disposed in association with the secondary magnet 138. The unbound target cells flow through the second processing chamber 76 and to the product receptacle 156. However, any paramagnetic beads which escape magnetic capture in the primary chamber 24 and which flow to the second processing chamber 76, are magnetically captured at this chamber. Consequently, the product receptacle 156 receives buffering solution which is rich in target cells, and which is substantially free of non-target cells and paramagnetic beads.

Now the machine will again instruct the operator to add fresh buffer solution to the chamber 24, and will release the magnetically captured paramagnetic beads, along with any remaining target cells which have been caught in the aggregations of beads. This is followed by a brief period of agitation. This release of the captured paramagnetic beads and period of agitation distributes into the fresh buffer solution substantially all of the target cells which may have been retained in the primary chamber 24. These activities are indicated generally by reference numeral 266 on Figure 9.

Subsequently, the machine 12 recaptures the paramagnetic beads in the primary processing chamber 24. However, because at this time the concentration of target cells in the buffering solution is now much lower, very few target cells will be caught in the aggregations of paramagnetic beads. The machine 12 again signals the attendant to drain the primary processing chamber 24 into the product receptacle 156 via the second processing chamber 76. These activities are generally indicated at numeral 268 on Figure 9.

At this time, the machine 12 enters a shut down sequence, indicated with the numeral 270 on Figure 9, during which it prompts the human attendant to properly close the disposable apparatus set 14, remove the set 14 from the machine 12, and dispose of the set 14. At this juncture, the operator can either shut off the machine 12, or command a reset, preparing the machine for another subsequent magnetic cell separation process (indicated with numeral 272 on Figure 9).

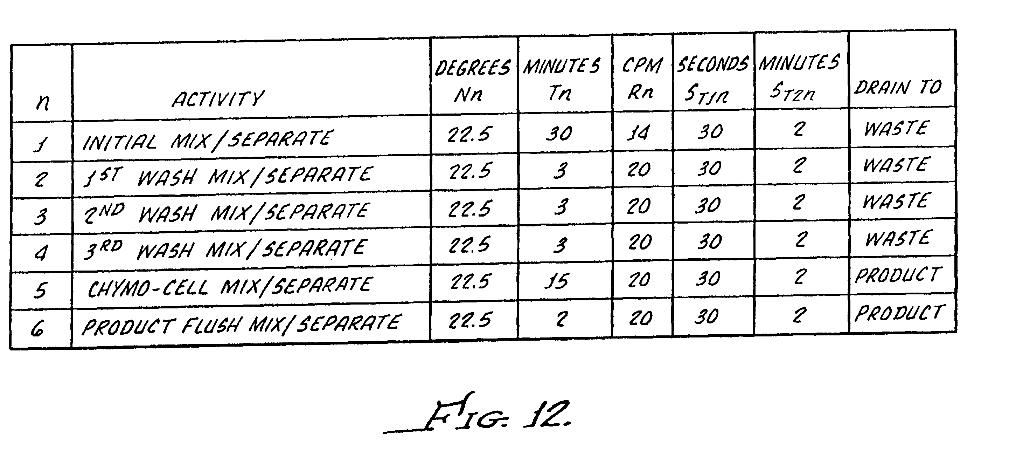

Considering now Figures 10-17, and keeping Figure 9 also in mind, the detailed steps in the method of operation of machine 12 with disposable apparatus set 14, and in conjunction with the occasional attentions of a human operator are set out. In these drawing Figures, several inputs into the process are referred to as "Attendant's Duties # _" (abbreviated as "AD # _"), these duties of the human operator are in each case prompted by the annunciator screen 148 of the machine 12, and require the human attendant to indicate to the machine 12 via an input at key pad 150 that each of the required actions has been completed. Accordingly, these "Attendant's Duties" inputs to the process in fact are the result of an annunciated request appearing on the screen 148, and an answering response from the human attendant. The machine 12 provides these annunciated requests, or prompts, as part of its programming. Although each of these activities is describe below in terms of the steps performed by the human attendant, each represents an output activity by the machine 12, to which the human attendant merely responds, and verifies to the machine 12 that the activity has been completed.

One of the significant aspects of the distinction outlined above is that the human attendant need never learn the steps in the magnetic cell separation process. Regardless of whether the human attendant has learned the complete process, the human attendant may have other duties also requiring this human's attention, such as a bank of machines 12 each at a different stage in a cell separation process, so that the human would find it very burdensome to remember where in the process each machine was at a particular time. The machine 12 tracks the complete magnetic cell separation process which it is performing, and this process may be varied according to the wishes of a supervisory person, such as the doctor for whom the cell separation is being performed, in order to maximize the yield of the process, for example. Accordingly, each "Attendant's Duties", input to the process represents a response to a corollary output prompt by the machine 12, to which the human attendant must also provide a verification input to the machine.

Viewing Figure 10, it is seen that the machine preparation sequence (252) first includes positioning by the machine 12 of the rocker assembly 126 in the vertical position seen in Figures 1 and 4; and retraction of the primary magnet assembly 400 composed of primary permanent magnets 202 to the position seen in Figure 4 (indicated on Figure 10 with the numeral 274). These steps prepare the machine 12 for the operator to install on the machine 12 (as seen in Figure 1) the disposable apparatus set 14, as seen in Figure 2. The machine 12 prompts the operator: to close all clamps on the set 14; to connect the bags 118 and 120 to the set 14; to install the disposable apparatus set 14 on the machine 12; to open the vent clamp 30; to prime the set 14 (by admitting a small amount of buffer from bag 120 through all portions of the connecting tubing and into the primary chamber 24); to add the cell preparation from bag 118 into the primary chamber 24; to add the paramagnetic beads into the primary chamber 24; to add the blocking agent, if desired; to add a calculated amount of the buffer solution to bring the liquid level in chamber 24, or the concentration of cells in this liquid, to a desired level; to close all clamps on the set 14; and to close the vent clamp 30; and finally to check the setup of the apparatus 14 on the machine 12.

As pointed out above, the machine 12 prompts the operator to perform each of these steps individually, and the operator verifies each step to the machine 12 by making an appropriate entry on the key pad 150 before the next prompt is displayed. These attendant's duties are collectively referred to and abbreviated as, "AD # 1" on Figure 10, and are referenced with the numeral 276. At 278 on Figure 10 is indicated that the mechanical part of machine 12 is waiting while the operator is prompted, performs, and verifies to the machine by an operator input at the key pad 150 (abbreviated as, "OPI", and referenced with the numeral 280 on Figure 10) the performance of each of the operator's duties. The controller 182 of the machine 12 provides these prompts at screen 148, and receives the verifications at key pad 150.

When the operator has performed and verified the last one of the duties for AD # 1, the machine 12 under the control of controller 182 begins the initial mix and separation phase (254) of the magnetic cell separation process. This initial mix and separation phase includes the first of several mixing operations (indicated with numeral 282), and the first of several magnetic separations (magnetic capturing of the paramagnetic beads in primary chamber 24), indicated on Figure 10 with the numeral 284, which will be performed by the machine 12 during each magnetic cell separation process.

Viewing Figure 10 it is seen that the mixing and separating processes are each indicated as subroutines (indicated with the numerals 286 and 288, respectively), merely for purposes of convenience and brevity of the following description. Those ordinarily skilled in the programming arts will realize that current technology in high-speed microprocessor-based control systems, with large amounts of relatively low-cost programmable memory, suggests that a program simply include repeated lines of code for repeating similar or identical activities. The entire program is then executed serially, rather than effecting an interrupt of the program while a sub-routine is called, executed, and a return to the program is effected. However, this structure for the program may lead to a greater number of code lines, and would be very burdensome to explain for the present purposes. Accordingly, for the present purpose it should be recognized that the machine 12 will identically or substantially repeat some activities several times during a complete cell separation process. The repeated activities can be understood quickly by reference to a complete description of the activity when it is first performed. When the activity is substantially, but not identically repeated, only the differences are described.

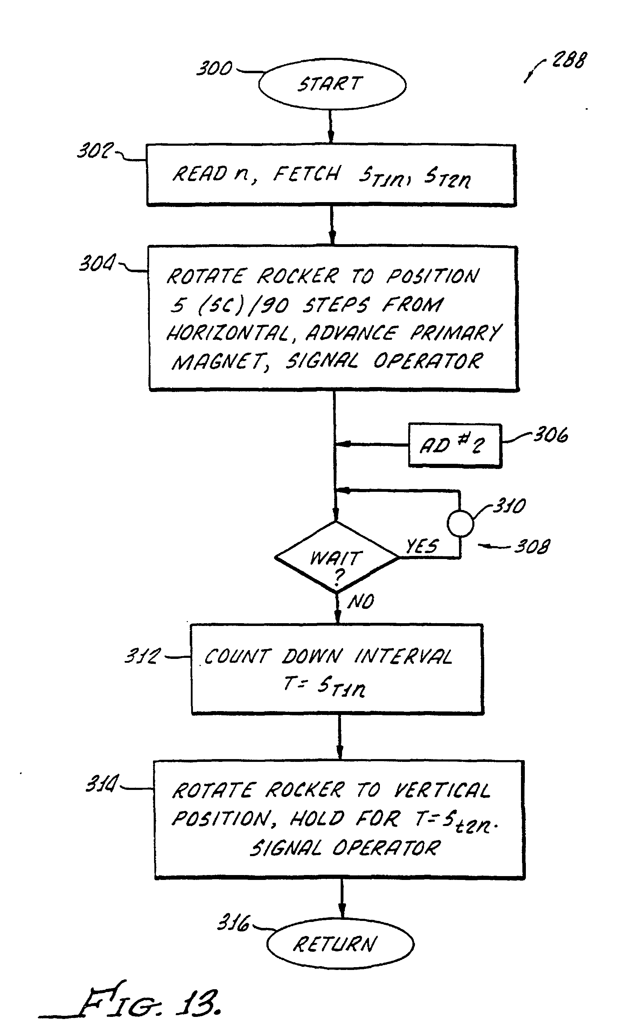

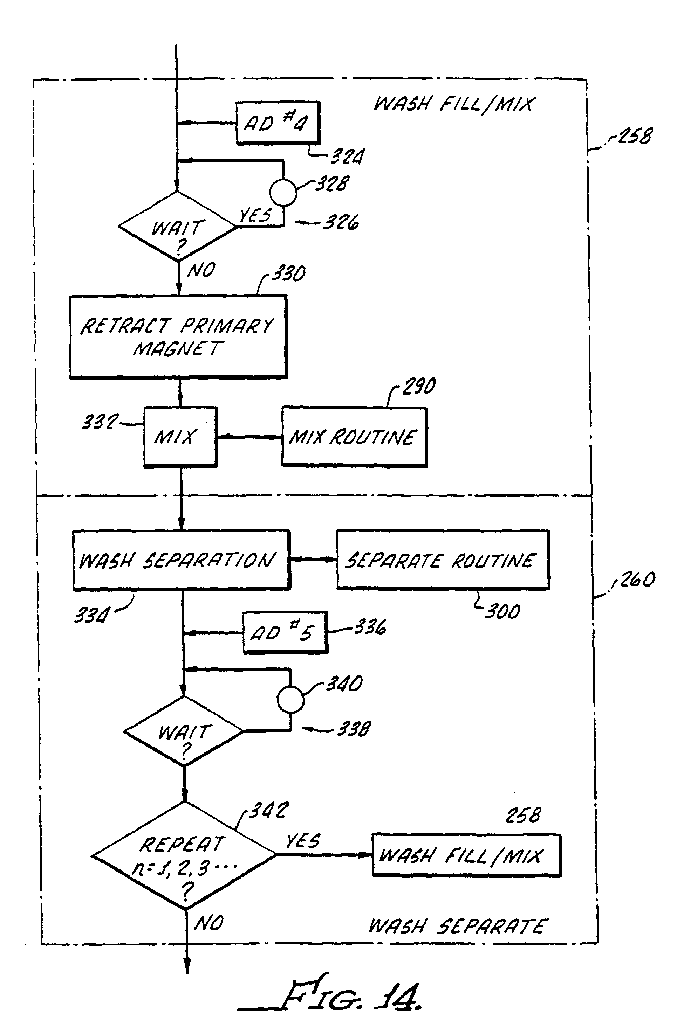

Keeping the above in mind while viewing Figures 11-13, the mix routine (286) is set out first in Figure 11. The mix routine 286 starts as is indicated at 290; and as indicated at 292, includes reading a value **n**. This **n** value tells the routine whether the mix to be performed is the first one, or one of the subsequent similar (but not necessarily identical) mixing operations performed by the machine 12 during a cell separation process. For the initial mix/separation, the **n** value is "1". A look-up table (Figure 12) provides the appropriate values for the mix routine. Nₙ, is the number of degrees in each direction from the horizontal through which the rocker assembly 126 is to be oscillated in order to agitate the contents of the primary chamber 24. Tₙ, is the time in minutes during which the agitation of the contents of the primary container 24 is to be continued by oscillation of the rocker assembly 126. Rₙ, is the rate in cycles per minute for the oscillation of rocker assembly 126. In other words, the rocker assembly 126 can be vigorously shaken back and forth, or gently tipped back and forth, and any intermediate rate of mixing is possible for the contents of the chamber 24, as desired.

As is indicated at 294, in order to implement the agitation or mixing of the contents of chamber 24, the machine 12 first rotates the rocker assembly 126 from the vertical position of Figures 1 and 4, to the horizontal position, which is indicated by the slit 226 aligning with sensor 178. Next, as is indicated at 296, the controller 182 begins to oscillate the rocker assembly 126 back and forth in each opposite direction α number of steps for motor 170 from the horizontal position. The α value is obtained by dividing the Nₙ value by the 0.6 degree (full step), or 0.3 degree (half step) incremental values for operation of the motor 170, and setting the closest integer value to the result equal to α. The oscillation of rocker assembly 126 begins in the clockwise direction, viewing Figure 1, because the rocker assembly is first moving in this direction away from the vertical position. The rate of oscillation is controlled by reference to the value Rₙ, and continues for the period Tₙ. At the end of interval Tₙ, the mix routine returns to the program seen in Figures 9 and 10.

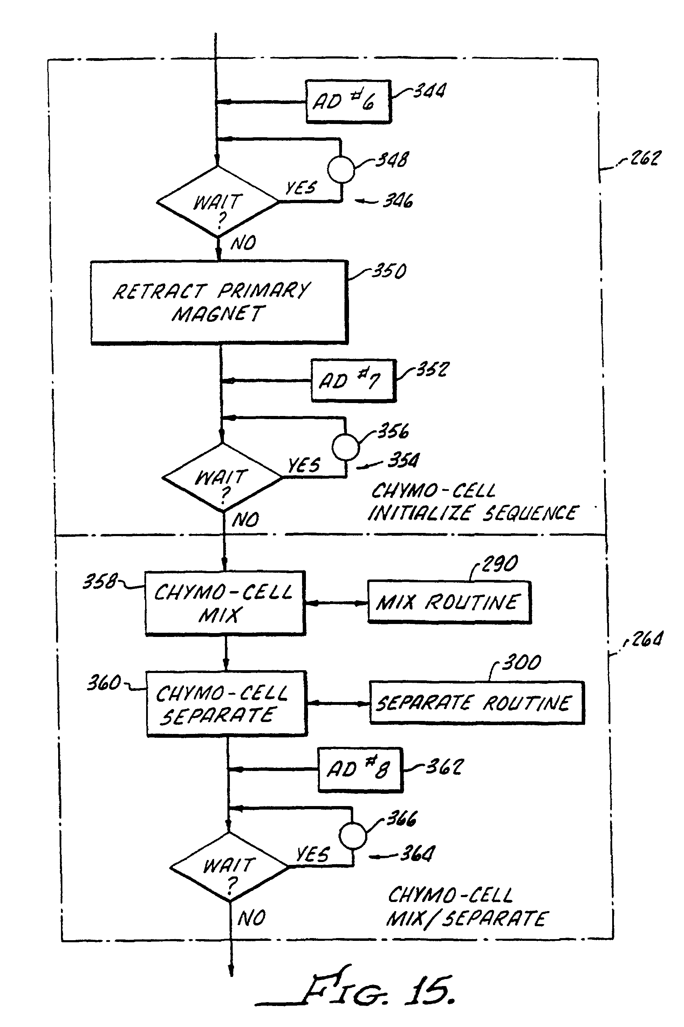

Immediately, the program calls the separate routine (288), which is seen in Figure 13. As indicated at 300, the separate routine starts, and immediately reads (indicated at 302) the **n** value, and fetches values Sₜ₁ₙ, and Sₜ₂ₙ, from the look-up table (Figure 12). The separate routine 288 continues pivotal movement of the rocker assembly in what ever direction it happened to be moving at the end of the time interval Tₙ for the mix routine, and brings the rocker assembly to a smooth stop at a programmable first separation position, which is preferably 5 degrees counter clockwise (viewing Figure 1, and as is shown in Figure 6) from the horizontal position for container 16 (this activity is indicated on Figure 13 with the numeral 304). In order to find this first position for the rocker assembly 126, the controller 182 pivots the assembly 126 counter clockwise from the horizontal position an integer number of steps for motor 170 which is most nearly equal to a value of 5 degrees divided by the 0.6 degree per step value explained above.

The primary magnet assembly 400 composes of permanent primary magnets 202 is then advanced to the position seen in Figure 6, and the operator is signaled. At this time, the operator is prompted (indicated as AD # 2, referenced on Figure 13 with the numeral 306) to add a small amount of buffer solution from bag 120 into the chamber 24, and to re-close the clamps of set 14. This addition of a small amount of buffer solution is effective to move back into the primary chamber 24 any target cells and paramagnetic beads which may have found their way into the tube 34 during agitation of the primary container 16. While the operator is performing this addition of buffer to chamber 24, the controller 182 waits, as is indicated with the numeral 308 on Figure 13.

When the operator indicates that AD #2 has been completed by making an appropriate operator input (OPI) at key pad 150, as is indicated at numeral 310 on Figure 13, then the controller 182 counts down a time interval in seconds equal to the value of Sₜ₁ₙ, as is indicated on Figure 13 with the numeral 312. This first separation position is held for the programmable time interval Sₜ₁ₙ, which is preferably 30 seconds. The applicants have determined that the initial time interval of 30 seconds is sufficient with the influence of the combined gravitational and magnetic precipitation of the bound target cells toward the underlying wall of the primary container 16 to magnetically capture greater than 90 percent of the bound target cells.

At this time, and if desired, the operator may also be prompted to drain a small amount of the fresh buffer solution which stays in tube 34 after the addition of this fresh buffer to chamber 24. This draining of a small amount of the fresh buffer is effected by opening the clamp 44, and allowing just a little buffer to drain to the waste container. While not essential to the performance of the magnetic cell separation process, this small draining operation is effective to clear from the Y-connector 36 any paramagnetic beads or non-target cells which may reside in the drain tube 40 above the clamp 44.

Next, as is indicated at numeral 314 on Figure 13, the controller 182 pivots the rocker assembly 126 to the vertical position seen in Figure 1, with the primary magnet assembly 400 kept in the advanced position. The controller 182 then counts down an additional separation time interval in minutes equal to the value Sₜ₂ₙ from the look up table of Figure 12. During this second phase of the two-phase magnetic separation of the present invention, the remaining bound target cells in the chamber 24 are captured in aggregations 234, recalling Figure 6. This time interval is preferably 2 minutes. At the end of the time interval Sₜ₂ₙ, the controller 182 signals the operator, and then executes a return to the main program of Figures 9 and 10, as is indicated at numeral 316 of Figure 13.

Returning to the program listing of Figure 10, and as is indicated at numeral 318 on this Figure, the operator is now prompted to perform a series of activities (attendant's duties # 3), which include: opening the drain clamp 44; opening the vent clamp 30; draining the liquid from chamber 24 into a waste container (the controller 182 obtains the "waste", or "product" destination for the liquid in chamber 24 from the look-up table of Figure 12); and re-closing the drain clamp. While the operator is performing these duties, the controller 182 waits, as is indicated at numeral 320 viewing Figure 13. When the operator has completed the duties for AD # 3, and has made the appropriate verification entries on the key pad 150, as is indicated with numeral 322 on Figure 13, then the controller 182 advances to the first wash fill/mix, as is indicated at 256 of Figure 9.

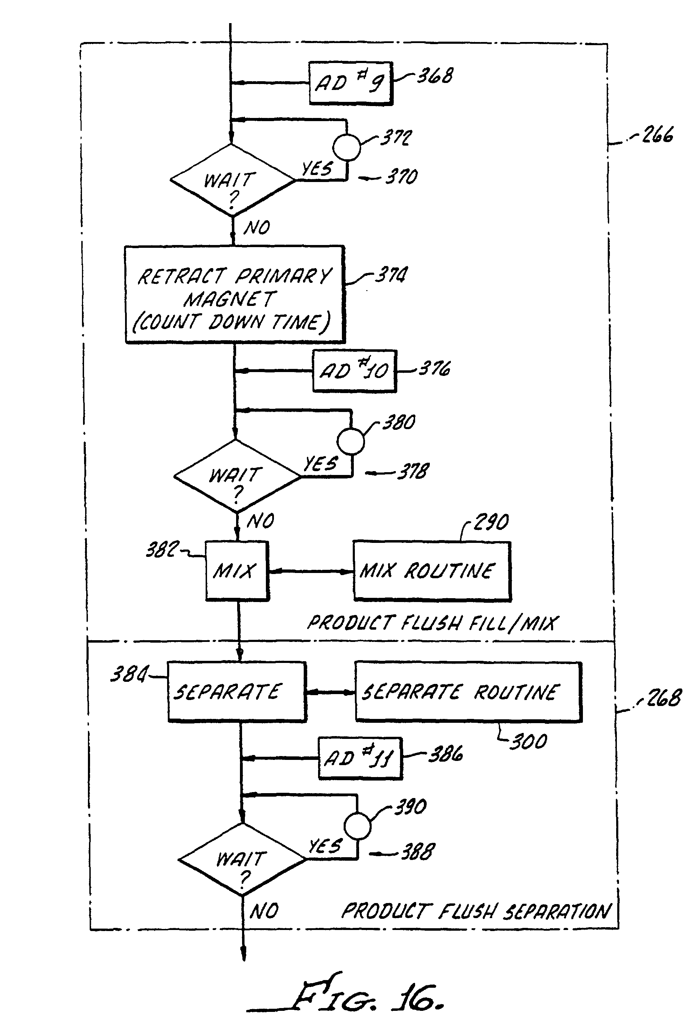

As is indicated at numeral 324 of Figure 14 (AD # 4), the operator is first prompted to: open the buffer clamp 66 and refill chamber 24 to level "A" with fresh buffer solution; to re-close all clamps; to re-close the vent clamp 30; and to verify the set up of the apparatus 14. Again, the controller 182 waits while displaying this sequence of prompts (indicated with numeral 326 on Figure 14), and when the operator has entered the last verification (indicated at numeral 328 of Figure 14), then the controller 182 retracts the primary magnet assembly 400 (this activity is indicated with numeral 330 on Figure 14), and increments the **n** value by one upon entering the next "mix" operation (numeral 332 on Figure 14). This mix operation calls the routine 290, which is repeated with the values obtained from the table of Figure 12. Thus, each mixing operation will be understood to be an individual event, the preferred values for which are shown in the table of Figure 12, but which values may be varied according to the wishes of the user of machine 12.

Next, the controller 182 enters the wash separation activity (refer to numeral 260 of Figure 9), calls the separation routine 300, and executes this routine with the appropriate values from the table of Figure 12 according to the current **n** value. It should be noted that only the initial mix separation of reference numeral 254 seen in Figure 9, and discussed with reference to the AD # 2 of Figure 13 includes the draining of a small amount of buffer solution from the Y-connector 36. If this option is included in the process, the controller 182 prompts the operator to perform this draining operation only when **n** = 1, and omits this draining operation from the operator prompts for all subsequent uses of the separation routine.

As is indicated at numeral 336 of Figure 14, the operator is now prompted to perform duties (AD # 5), which are the same as those for AD # 3, numeral 318, discussed above with reference to Figure 10. Again the controller 182 waits (numeral 338 of Figure 14), and must receive a series of verifications (numeral 340 on Figure 14) before proceeding to the next phase of the magnetic cell separation process.

As is indicated with numeral 342 on both Figures 9 and 14, the supervisory user of the machine 12 has an opportunity to enter a value for a conditional branching decision. This conditional branching decision allows the wash fill/mix, and wash separation steps to be conducted only once, or to be repeated. Preferably, the wash fill/mix, and wash separation steps (258, 260) are repeated three times. However, dependent upon the preferences of the user of the machine 12, these steps may be repeated only twice, or may be repeated more than three times. Attention to the look-up table of Figure 12 will show that this exemplary table includes values for three identical wash fill/mix, and wash separation steps. However, the supervisory user of the machine 12 may enter different values into the table for the steps 258, 260, and may have these steps repeated more than three times simply by indicating this preference at the conditional branching 242, and entering the desired values in table 12 if the preferred default values of the look up table of Figure 12 are not to be used. Alternatively, the machine 12 may be programmed to always perform three washings, even though the operator may be given an opportunity to alter the rate, time interval, and other parameters of these washings. Still alternatively, the machine 12 may be provided with a manual mode in which the operator may enter activities and parameter values for these activities at the key pad 150.

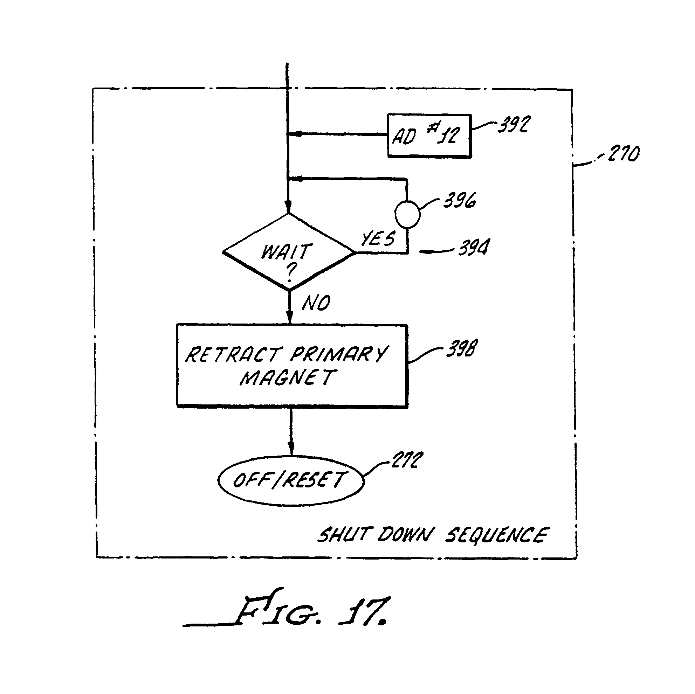

Next, the controller 182 enters the releasing material initialize sequence (numeral 262 of Figure 9). At this time the operator is prompted to perform duties (AD # 6, indicated with numeral 344 on Figure 15), which include: filling the chamber 24 to level "B" with fresh buffer solution; re-closing the buffer clamps; and adding the enzyme material to the chamber 24 via the port 32. As before, the controller 182 waits (indicated with numeral 346 on Figure 15) while the series of prompts are displayed, and the operator verifies (numeral 348 of Figure 15) that each activity has been completed. The controller 182 then retracts the primary magnet assembly 400, as is indicated at 350 of Figure 15, and prompts the operator to perform AD # 7. This activity simply involves the operator closing the vent clamp 30, and checking the set up of the apparatus 14. Again, the controller 182 waits (354) for verification (356) from the operator that AD # 7 is completed.

As is indicated at numerals 358 and 360 of Figure 15, the controller 182 then conducts another mixing activity followed by another separation activity, with the value of n incremented again so that the appropriate values from table 12 are selected. These mixing and separating activities are indicated at Figure 9 with the numeral 264. At the end of the releasing material separation activity, as is indicated by numeral 362 of Figure 15, the operator is prompted to perform AD # 8. This set of attendant's duties includes: the operator preparing (and, if necessary, connecting) the product container 156; opening the appropriate one of the product clamps 64, 66 (recalling Figure 2); opening the vent clamp 30; and draining the liquid product along with the target cells from chamber 24 into the product container 156 via the chamber 76 of the secondary container 68. At the completion of draining of chamber 24, the operator is also prompted to re-close the product clamp previously opened. Again, the controller 182 waits (364), and must obtain verification of completion of each activity (366), before moving on in the process.

Now the controller 182 enters the product flush fill/mix, and product flush separation steps (266, and 268, respectively), as set out in Figure 9. As is seen at numeral 368 of Figure 16, the operator is prompted by the controller 182 to perform AD # 9. This set of attendant's duties requires the operator to: refill the chamber 24 with fresh buffer solution to level "A"; and re-close the buffer clamps. The controller 182 waits (370), and requires verification (372) of AD # 9 being completed before going on in the process.

During the performance of AD # 9, while the operator is adding buffer solution to the primary chamber 24, the controller 182 retracts the primary magnet assembly 400. Preferably, the retraction of the primary magnet assembly 400 occurs about 30 seconds into the refilling of the chamber 24 with buffer solution. This retraction of the primary magnet assembly 400 after the refilling of the primary chamber 24 is under way, but before this refilling is completed has two important advantages. First, the partial filling of chamber 24 with buffer solution provides a liquid cushion so that when the target cells and paramagnetic beads are released they do not fall against the bottom wall 22 of the container 16. Secondly, by continuing the addition of buffer solution after the magnetic release of the target cells and paramagnetic beads by retraction of the primary magnet assembly 400, the inflow of liquid buffer solution is effective to assist in separating target cells from the paramagnetic beads, and in breaking up the aggregations of these beads which could trap target cells. This retraction of the primary magnet assembly 400 is commanded by the controller 182 a selected time interval (preferably about 30 seconds) after the operator supplies one of the required operator inputs (OPI's) of AD # 9, which input indicates that refilling of chamber 24 with buffer solution has begun.

Following the completion of the AD #9 duties, and the retraction of the primary magnet assembly 400, the controller requires the operator to perform AD # 10 (indicated with numeral 376 on Figure 16), also waiting while this activity is performed (numeral 378 of Figure 16), and requiring verification (numeral 380) that the activity is completed. The activity for AD # 10 simply requires the operator to: re-close all clamps; to re-close the vent clamp 30; and to again check the set up of the apparatus 14. The controller 182 then mixes the contents of the primary container 16 (382, viewing Figure 16), using the mix routine 290, with the **n** value incremented by one so that the appropriate values are selected from the table of Figure 12.

Upon the completion of the product flush mixing (352), the controller 182 separates (384, viewing Figure 16) the paramagnetic beads onto the wall of the container 16 once again by use of the separate routine 300. This captures the paramagnetic beads in primary chamber 24, and allows the buffer solution to be again drained into the product container 156 with additional target cells. These activities are prompted at AD # 11 (referenced with numeral 386 on Figure 16), with controller 182 waiting (388), and requiring verification (390, on Figure 16) that this activity has been completed.

Finally, the controller 182 enters the shut down sequence, shown at 270 of Figure 9. Viewing Figure 17, it is seen that this shut down sequence includes a set of attendant's duties, AD # 12, referenced with the numeral 392 on this Figure. These attendant's duties include: closing all of the clamps on the disposable apparatus set 14; and removing the apparatus set 14 from the machine 12. As before, the controller 182 waits (394), and requires verification (396), that each activity for the attendant is completed. The operator will be trained to properly dispose of the set 14, and waste container 42a, treating them as hazardous biological waste. Next, the controller 182 retracts the primary magnet assembly 400 (indicated at 398) so that the operator can more easily install a new set of disposable apparatus 14 on the machine 12, if another magnetic cell separation process is to be performed presently. Now the operator may turn off the machine 12, or select a reset operation, which starts the entire program sequence and magnetic cells separation process over again, as was pointed out with respect to Figure 9 when this Figure was first considered above.

The practice of this invention is illustrated by the following experimental examples.

### EXAMPLE 1

### Isolation of CD34+ stem cells from bone marrow aspirates.

A concentrated low-density fraction of leukocytes, also known as hematopoietic mononuclear cells (MNC), was separated by conventional means from bone marrow aspirates of human patients and healthy donors. The cells in the samples were separated by centrifigation through Ficoll/Histopaque (Sigma) and the mononuclear cells from the interface band were collected (Smith, S.L., et al., Exp Hematol 21:870-877, 1993). Certain MNC samples were subjected, by the physician, to filtration through sterile gauze and/or stainless steel screens prior to administration of the bone marrow to the patient. For certain pre-clinical experiments, trapped MNC were recovered from the screens and/or gauze. For other experiments, MNC of unfiltered bone marrow aspirates were used.

Sensitization of cells: The target CD34+ cells in the MNC concentrate were "sensitized" with an anti-CD34 antibody. The anti-CD34 antibody, also known as "anti-My10", is described in U.S. Patent No: 4,965,204 (Civin) as the monoclonal antibody secreted by hybridoma #HB-8483 deposited with the ATCC, Rockville, MD. During the sensitization step, the anti-CD34 antibody specifically binds to the CD34 antigen on the target cells. The mononuclear cells were incubated with slow (4 rpm) end-over-end rotation for 30 minutes at 2 - 8°C with 0.5 µg of anti-CD34 antibody per 10⁶ cells [anti-CD34 #9069 prepared by serum-free cell culture (Baxter Hyland, Hayward, CA)]. The cells were then washed three times to remove residual unbound antibody.

Preparation of paramagnetic microspheres: Dynal® paramagnetic microspheres (beads) coated with sheep-anti-mouse antibodies were provided with the Isolex®300 kit at a concentration of 4 X 10⁹ beads in 10 ml of buffer. The Dynal® beads were washed three times to remove the buffer containing 0.2% sodium azide present in the bead suspension.

Mixing sensitized cells with Dynal® beads: The washed sensitized cells, the washed beads, and a concentrated preparation of human Ig (Gammagard®) were introduced into the primary chamber of the Isolex®300. (Gammagard® is a concentrate of human immunoglobulins which is added to reduce non-specific binding of cells.)

Operation of the Isolex®300: The Isolex®300 Magnetic Cell Separator prompted the operator to perform an on board 30 minute incubation, at room temperature, while mixing continuously. Beads clustered around each CD34+ cell forming a rosette by binding of the sheep-anti-mouse antibody on the bead surfaces to the mouse monoclonal anti-CD34 antibody bound to the stem cell.

Separation of beads/rosettes from non-target cells: After mixing, and according to the programmed operation of the Isolex®300, the beads/rosettes were magnetically held in the Isolex® primary chamber while the non-target cells were allowed to gravity flow out of the chamber into a pre-connected 1000 ml transfer pack.

Washing of beads/rosettes: After removing the non-target cells, three wash cycles were performed. The operator initiated the three wash cycles by following the prompts displayed on the instrument. The wash buffer was collected along with the non-target cells in the 1000 ml transfer pack. The contents of this pack were assayed to determine capture efficiency.

Release of cells from beads: The cells were released from the Dynal® beads by enzymatic digestion using ChymoCell-T™ (ChymoCell-T™ is a clinical grade chymopapain supplied by Boots, Ltd., U.K.). The ChymoCell-T™ was first reconstituted with sterile water provided in the Isolex®300 kit, and then introduced into the Isolex® primary chamber containing the bead/rosettes and buffer by aseptic injection through the injection site (final chymopapain concentration = 200 units/ml). The operator prompts elicited a 15 minute, room temperature incubation, with constant mixing. The beads were then magnetically retained in the primary container while the CD34+ target cells were passed over the secondary magnet and collected into a final collection container.

Phenotype analysis of cell populations: The cells released from the beads were then evaluated for CD34+ purity by staining with the FITC-8G12 monoclonal antibody to CD34 (Baxter Immunotherapy Division, Irvine, CA) for 15 minutes on ice and quantitated with a FACSan® flow cytometer (Becton Dickenson, San Jose, CA) as described (Smith, S.L., et al, Exp Hematol 21:870-877, 1993).

Results: The combined results from bone marrow aspirates (3) and bone marrow screen samples (5) are shown in Table 1 below.

**Table 1**

| | | |
|---|---|---|
| Starting CD34+ cells | 1.10 +/- 1.19 % | (range 0.16-3.48) |
| Final CD34+ cells | 73.0 +/- 19.5 % | (range 42.8 - 88.0) |
| % yield | 50.1 +/- 21.8 % | (range 18.0 - 79.7) |
| Fold enriched | 146 +/- 116 | (range 25 - 296) |

Individual results from 3 additional bone marrow aspirate samples are shown in Table 2 below.

**Table 2**

| Starting MNC | | | |
|---|---|---|---|
| Total cells | 9 X 10⁹ | 1 X 10¹⁰ | 1 X 10¹⁰ |
| % CD34+ cells | 1.56 | 2.12 | 1.4 |

| CD34+ Cell Isolate | | | |
|---|---|---|---|
| Total cells | 4.9 X 10⁷ | 1.09 X 10⁸ | 5.7 X 10⁷ |
| % CD34+ cells | 73.8 | 76 | 72 |

### Example 2

### Colony formation by isolated CD34+ cells.

Standard colony assays were performed to determine how many individual cells were capable of proliferating to form colonies of more differentiated cells. Briefly, 5 X 10⁴ cells (before separation) or 5 X 10² cells (after separation) were plated in each 35-mm dish in 1 ml media (Terry Fox Laboratory) containing IMDM, pre-screened fetal bovine serum, and methylcellulose. Colony stimulating activity was provided by G-CSF, GM-CSF, IL-3, IL-6, and stem cell factor (20 ng each per dish). CFU-GM (colorless cluster of 50 or more cells), BFUe (clusters of 50 or more red cells), and CFU-Mix (colonies containing both morphologies) were scored after 14 days in culture using an inverted microscope.

Results: Colony formation by cells from 2 isolation experiments were averaged (see Table 3 below).

**Table 3**

| | Colonies per 10⁴ cells | | |
|---|---|---|---|
| | CFU-Mix | CFU-GM | BFUe |
| Before selection | 14 | 33 | 9 |
| CD34+ selected | 713 | 3413 | 303 |
| Fold enriched | 51 | 104 | 56 |

### Example 3

### Selection of CD34+ cells from mobilized perpheral blood samples.

Donors were treated with G-CSF (granulocyte-colony stimulating factor), or G-CSF plus GM-CSF, which treatment stimulates the "mobilization" of stem cells from the bone marrow to the peripheral blood, thus greatly increasing the numbers of stem cells obtainable from a peripheral blood sample.

Leukocytes were collected from donors and CD34+ cells were isolated as described in Example 1 above. Cell populations were analyzed for CD34+ expression by FACS as described above.

Results: The results from 17 experiments are shown in Table 4 below.

**Table 4**

| | %CD34+ | % Yield |
|---|---|---|
| MNC pre-selection | 0.47 +/- 0.33 (range 0.4-1.5) | |
| Isolated CD34+ cells | 73.2 +/- 10.7 (range 58.3-92.0) | 40.8+/-16.1 (range 16.4-67.5) |

### Example 4

### Colony formation by CD34+ cells isolated from peripheral blood.

Cell populations originating from mobilized peripheral blood (Example 3 above) were assayed for colony formation potential in standard methylcellulose assays (Example 2 above).

Results: Results from three isolation experiments are averaged in Table 5 below.

**Table 5**

| | Colonies per 10⁴ cells | | |
|---|---|---|---|
| | CFU-Mix | CFU-GM | BFUe |
| Before selection | 12 | 15 | 5 |
| CD34+ selected | 575 | 2366 | 365 |
| Fold enriched | 54 | 156 | 89 |

### Example 5

### Depletion of CD3+ cells (T-cells) from CD34+ cell isolates.

It is desirable to deplete T-cells from bone marrow intended for allogeneic transplant. Certain T-cells in donated bone marrow are thought to be partially responsible for Graft-Versus-Host Disease, in which the lymphocytes in the transplanted cell population mount an immune reaction against the tissues of the recipient. The cell surface antigen CD3 is specific to T-cells, and can be used as a marker to estimate the numbers of T-cells in a graft population.

Bone marrow samples were processed on the Isolex®300 as described in Example 1 above to select CD34+ cells.

An older method to deplete T-cells involves selective precipitation of CD3+ cells by the erythrocyte rosette/soybean agglutinin method (E rosette/SBA) (Frame, J.N., et al., 1989, Transplantation 47:984-8; Reisner, Y., et al, 1981, Lancet 2:327-31; Reisner, Y., et al., 1980, Lancet 2:1320-4). This method generally requires 16-18 hours of technical attention for completion.

Bone marrow samples before and after CD34+ selection on the Isolex®300 were assayed for CD3 antigen by FACS analysis. Likewise, bone marrow samples depleted of CD3+ cells by the E rosette/SBA method were assayed for CD3 antigen by FACS analysis.

Results: The results from 5 bone marrow samples are averaged in Table 6 below.

**Table 6**

| | CD3+ cells/kg | log depletion |
|---|---|---|
| CD34+ cells/Isolex®300 | 2.2 X 10⁴ | 2.73 +/- 0.28 |
| E rosette/SBA depleted cells | 9.86 X 10⁵ | 1.41 +/- 0.35 |

### CONCLUSION

Selection for CD34+ cells on the Isolex® provides an enriched population of stem/progenitor cells which are capable of proliferation into more differentiated hematopoietic cells. Moreover, undesired T-cells are depleted from the population by positive CD34+ selection on the Isolex®.

### EXAMPLE 6

### Hematopoietic Potential of CD34+ Enriched Autologous Bone Marrow Grafts.

Bone marrow harvests from breast cancer patients were processed to produce a suspension of mononuclear cells (MNC) as described in Example 1. Two portions of the harvested MNC were cryopreserved for MNC transplantation and backup (1 X 10⁸ cells/kg each), while the remainder was processed for CD34+ selection using the Isolex®300 immunomagnetic selection method of the invention as described in Example 1. The selected CD34+ cells were subsequently cryopreserved. Of eight bone marrow samples processed, purity of CD34+ cells in the selected product ranged from 56-89% (mean=77%). Selected cells were 47 to 72 fold enriched in CD34+ cells (mean=59) and represented a 30-72% recovery of Cd34+ cells (mean=48). Contaminating CD34 negative cells in the product consisted primarily of CD19+ B cells (22-27%), with very few (1-8%) CD3+ T cells.

The progenitor recovery and hematopoietic potential of the CD34-enriched cell grafts infused into four patients was evaluated in in vitro progenitor assays and in long-term hematopoietic cultures (LTHC). CD34 selected cells, which were enriched 31-68 fold in their content of total progenitors (mean=53 fold) yileded 89% of CFU-GM as well as total progenitors present in MNC and provided 8.7 to 12.4 X 10⁴ total progentitors/kg (mean=10.8 X 10⁴). In LTHC initiated with freshly-isolated or cryopreserved CD34-enriched cells, progenitor production continued for up to 43 days, yielding up to a 12-fold increase in numbers of CFU-GM. Patients received 3.8 to 10 X 10⁵ CD34+ cells/kg (mean=6.7 X 10⁵) in grafts of CD34-enriched cells, and engrafted with a median time to an absolute granulocyte count 500/L in 19 days (range 15-21 days). There were no apparent differences in the rate of immune reconstitution of any lymphohematopoietic lineage following transplantation with either cryopreserved MNC or cryopreserved CD34-enriched bone marrow cells. These data suggested that CD34-enriched cells isolated by the method of the invention retained their full hematopoietic potential and provided suitable stem cell grafts for patients undergoing autologous bone marrow transplantation.

### EXAMPLE 7

### Enaraftment With Autologous Bone Marrow Transplantation With Either Full Bone Marrow or Selected CD34+ Cells.

Breast cancer patients undergoing tandem high dose chemotherapy with autologous bone marrow transplant (ABMT) were randomized to receive full bone marrow or CD34+ enriched cells in either cycle one or cycle two. Patients with high risk Stage II, IIIB, or previously untreated stage IV breast cancer underwent bone marrow harvesting of 2-4 X 10⁸ nucleated cells/kg. The harvest was divided into 3 portions: 1 X 10⁸ cells/kg for one cycle of therapy, 1 X 10⁸ as backup, and the remainder was processed for CD34+ selection as described in Example 1. Patients then received the first of two planned cycles of cyclophsphamide, carboplatin, and etoposide, followed by infusion of either full bone marrow or CD34+ selected cells on alternate cycles. G-CSF was given post transplant. In four patients, the median number of cells harvested was 3.94 X 10⁸ nucleated cells/kg (range 2.63 - 7.01). The median number of CD34-enriched cells infused was 8.61 X 10⁵ MNC/kg (range 6.35-18.0). The median percentage of CD34+ cells in the full bone marrow harvest was 1.13% (range 0.82-1.38%) and that in the selected cells was 63.4% (range 56.9-73.8%). All patients engrafted with a median time to an absolute granulocyte count 500/µl of 15 days (range 10-20 days) for cycles in which full bone marrow was given and 19 days (range 15-21 days) for cycles with CD34 selected cells. Time to platelet engraftment: 38 days (range 30-43 days) for bone marrow, 29 days (range 27-34 days) for CD34 selected cells. Length of hospitalization for bone marrow cycles: 24 days (range 22-32 days); for CD34 selected cells: 26 days (range 25-29 days). The numbers of packed red blood cell transfusions and platelet transfusions required were comparable between the two treatments. In conclusion, CD34+ selected cells provided engraftment comparable to that of full bone marrow engraftment. Moreover, the infusion of CD34+ selected cells may provide an advantage in reduction of tumor cell burden in the graft, and reduced volume of infused cells. Along with a reduced volume of infused cells, there is a reduction in volume of infused DMSO, which is used as a cryopreservative prior to cell thawing and infusion.

While the present invention has been depicted, described, and is defined by reference to a particularly preferred embodiment of the invention, such reference does not imply a limitation on the invention, and no such limitation is to be inferred. The invention is capable of considerable modification, alteration, and equivalents in form and function, as will occur to those ordinarily skilled in the pertinent arts. The depicted and described preferred embodiment of the invention is exemplary only, and is not exhaustive of the scope of the invention. Consequently, the invention is intended to be limited only by the spirit and scope of the appended claims, giving full cognizance to equivalents in all respects.

## Claims

1. Apparatus for magnetic cell separation using paramagnetic microbeads, said apparatus comprising:
a base member, said base member movably supporting an agitation member, and first power drive means for agitating said agitation member relative to said base member;
said agitation member including means for removably securing thereto a primary separation container within which a liquid suspension of the cells and microbeads may be agitated by movement of said agitation member; said agitation member further movably carrying a primary magnet for movement between a first position adjacent to the secured primary container and in which magnetic flux from said primary magnet penetrates the primary container to attract and capture the microbeads, and a second position spaced from the primary container so that said magnetic flux does not capture the microbeads; and second power drive means for selectively moving said primary magnet between said first and second positions independently of agitation of said primary container; and
further including said base member carrying a holder for a secondary container fluidly in communication with said primary container, and a secondary magnet disposed adjacent to said secondary container for capturing paramagnetic microbeads which escape from said primary container.

2. The apparatus of Claim 1 wherein said base member pivotally carries said agitation member and is configured to secure an elongate primary container thereto, said first power drive means also being effective to tiltingly move said agitation member and primary container between a first position in which the primary container is vertical, and a second position in which the primary container is horizontal, and is agitated by said first power drive means effecting successively opposite tilting movements of said agitation member and primary container from said horizontal second position.

3. The apparatus of Claim 2 wherein said first power drive means is effective to also move said agitation member and primary container to a first separation position in which said agitation member and the primary container are angulated relative to said horizontal second position by a certain angle.

4. The apparatus of Claim 3 wherein said certain angle is substantially 5 degrees.

5. The apparatus of Claim 3 wherein said apparatus further includes an operator input device by which said human operator can make an input to said microprocessor-based controller.

6. The apparatus of Claim 3 further including sensor means for indicating to said control means when said agitation member is in said first position and when said agitation member is in said second position, said sensor means including an encoder disk rotating with said agitation member, said encoder disk having at least one feature which is distinguishable from the remainder of said encoder disk, and at least one sensor device responsive to said at least one feature and providing an input to said control means in response to alignment of said feature with said sensor device.

7. The apparatus of Claim 6 wherein said encoder disk has a first feature which aligns with said sensor device when said agitation member is in said first position, and a second similar feature which aligns with said sensor device when said agitation member is in said second position.

8. The apparatus of Claim 3 further including sensor means for indicating to said control means when said primary magnet is in said first position, and when said primary magnet is in said second position.

9. The method of using paramagnetic microbeads to perform a magnetic cell separation which includes multiple activities in sequence, said method comprising the steps of:
providing a machine which is programmed with said multiple activities and their sequence;
using said machine to perform plural certain ones of said multiple activities in sequence;
using said machine to prompt a human operator to perform plural selected ones of said multiple activities in sequence; and
requiring said human operator to make a verification input to said machine upon the completion of each individual one or a group of said plural selected activities;
causing said machine to automatically perform one of said certain activities of said multiple activities in said magnetic cell separation upon the receipt by said machine of a respective verification input from said human operator that an immediately preceding one of said selected activities has been completed;
using said machine to summon said human operator when said machine has completed one of said certain activities in preparation for said human operator performing the next sequential one of said plural selected activities;
using said machine to perform the certain steps of: agitation of a primary container containing a mixture of cells and paramagnetic microbeads in liquid; magnetic capture of said paramagnetic microbeads in said liquid; and magnetic release of said paramagnetic microbeads in said liquid;
providing said machine with an agitation member which carries said primary container and which is movable to effect said agitation;
providing on said agitation member a primary magnet which is movable between a first position adjacent to said primary container to effect said magnetic capture of said paramagnetic microbeads and a second position spaced from said primary container to effect magnetic release of said paramagnetic microbeads; and
moving said primary magnet by power drive means between said first and said second positions independently of agitation of said primary container by movement of said agitation member.

10. The method of Claim 9 additionally including the steps of causing said machine to perform an initial agitation of said primary container to bond selected target cells from said mixture to said paramagnetic beads, followed by an initial magnetic capture of said paramagnetic beads and bound target cells in said primary container;
using said machine to prompt said operator to drain said liquid from said primary container to waste, and to add fresh liquid to said primary container;
causing said machine to magnetically release said paramagnetic beads into said fresh liquid within said primary container;
next causing said machine to agitate said primary container with said fresh liquid and target cells bound to said paramagnetic beads, and to magnetically recapture said paramagnetic beads and bound target cells;
using said machine to prompt said operator to add a material to said primary container which will unbind said target cells from said paramagnetic beads, causing said machine to magnetically release said paramagnetic beads and bound target cells into said fresh liquid and added material therein, and using said machine to agitate said primary container to effect unbinding of said target cells from said paramagnetic beads;
causing said machine to recapture said paramagnetic beads in said primary container while said target cells remain mixed in said liquid, and using said machine to prompt said operator to drain said liquid and said target cells from said primary container to a product collection container;
including a secondary magnet on said machine;
including in said apparatus set a secondary container in juxtaposition with said secondary magnet;
flowing said liquid and said target cells to said collection container through said secondary container past said secondary magnet; and
using said secondary magnet to capture any paramagnetic microbeads which have escaped from said primary container.

11. The method of Claim 10 additionally including the steps of using said machine to prompt said operator to add fresh liquid into said primary container, and using said machine to: magnetically release said paramagnetic beads along with any entrapped target cells into said fresh liquid, to effect agitation of said primary container to separate any entrapped target cells from said paramagnetic beads, to magnetically recapture said paramagnetic beads while said target cells remain mixed in said liquid, and using said machine to prompt said operator to drain said liquid and target cells to said product container.

12. The method of Claim 11 additionally including the steps of using said machine to prompt said operator to close said apparatus, and to remove said apparatus from said machine.

13. The method of Claim 9 additionally including the steps of configuring said machine to tilt said primary container about a pivot axis to effect said agitation.

14. The method of Claim 13 further including the steps of causing said machine to tilt said primary container successively in opposite directions with respect to a horizontal position of said primary container in order to effect said agitation.

15. The method of Claim 13 also including the steps of providing for said machine to selectively position said agitation member by operation of said first power drive means, and causing said machine to tilt said agitation container to a first selected angulated position with respect to both the horizontal position and a vertical position of said agitation container to effect a first phase of said magnetic capture of said paramagnetic beads.

16. The method of Claim 15 wherein said first selected angulated position for said magnetic capture of said paramagnetic beads is selected to be substantially 5 degrees from said horizontal position.

17. The method of Claim 16 further including the step of retaining said primary container in said first selected angulated position for a selected interval of time following movement of said primary magnet to said first position thereof to effect said first phase of magnetic capture of said paramagnetic beads.

18. The method of Claim 17 also including the step of causing said first time interval to be substantially 30 seconds.

19. The method of Claim 18 additionally including the step of selectively tilting said agitation member to a vertical position for said primary container for a second phase of said magnetic capture of said paramagnetic beads.

20. The method of Claim 19 including the step of causing said second phase of said magnetic capture of said magnetic beads to have a time interval of substantially 2 minutes.

21. The method of Claim 10 additionally including the step of using said machine to prompt said operator to add an anticoagulant material to said primary chamber prior to said initial agitation and bonding of said target cells to said paramagnetic beads.

22. The method of Claim 10 also including the step of using said machine to prompt said operator to add a small amount of fresh liquid to said primary container following said initial agitation step and prior to said initial magnetic capture of said paramagnetic beads.

## Patentansprüche

1. Vorrichtung zur magnetischen Zelltrennung unter Verwendung von paramagnetischen Mikroperlen, wobei die Vorrichtung aufweist:
ein Basiselement, wobei das Basiselement beweglich ein Schüttelelement abstützt, und erste Kraftantriebsmittel zum Schütteln des Schüttle relativ zu dem Basiselement;
wobei das Schüttelelement Mittel zum abnehmbaren Sichern eines primären Trennbehälters an sich umfasst, in dem eine flüssige Suspension der Zellen und der Mikroperlen durch Bewegung des Schüttelelements geschüttelt werden kann; wobei das Schüttelelement weiterhin einen primären Magnet zur Bewegung zwischen einer ersten Position benachbart dem gesicherten primären Behälter, in der der magnetische Fluss von dem primären Magnet den primären Behälter durchsetzt, um die Mikroperlen anzuziehen und einzufangen, und einer zweiten Position beabstandet von dem primären Behälter, so dass der magnetische Fluss die Mikroperlen nicht einfängt, beweglich abstützt; und wobei zweite Kraftantriebsmittel zum selektiven Bewegen des primären Magnets zwischen der ersten und der zweiten Position unabhängig von dem Schütteln des Primärbehälters vorgesehen sind; und
wobei weiterhin vorgesehen ist, dass das Basiselement einen Halter für einen sekundären Behälter abstützt, der in Fluidkommunikation mit dem ersten Behälter steht, wobei ein sekundärer Magnet benachbart dem sekundären Behälter zum Einfangen von paramagnetischen Mikroperlen angeordnet ist, die aus dem primären Behälter entweichen.

2. Vorrichtung nach Anspruch 1, wobei das Basiselement das Schüttelelement schwenkbar abstützt und ausgebildet ist, um einen langgestreckten primären Behälter daran zu sichern, wobei die ersten Kraftantriebsmittel auch wirksam sind, um das Schüttelmittel und den primären Behälter zwischen einer ersten Position, in dem der primäre Behälter vertikal steht und einer zweiten Position, in der der primäre Behälter horizontal steht, zu verschwenken und wobei das Schüttelelement von den ersten Kraftantriebsmitteln geschüttelt wird, was aufeinander folgende entgegengerichtete Schwenkbewegungen des Schüttelelements und des primären Behälters aus der horizontalen zweiten Position bewirkt.

3. Vorrichtung nach Anspruch 2, wobei die ersten Kraftantriebsmittel wirksam sind, um das Schüttelelement und den primären Behälter auch in eine erste Trennposition zu bewegen, in der das Schüttelelement und der primäre Behälter relativ zu der horizontalen zweiten Position um einen bestimmten Winkel angewinkelt sind.

4. Vorrichtung nach Anspruch 3, wobei der bestimmte Winkel im wesentlichen 5° ist.

5. Vorrichtung nach Anspruch 3, wobei die Vorrichtung weiterhin eine Bedienereingabeeinrichtung umfasst, über die ein menschlicher Bediener Eingaben an die mikroprozessorbasierte Steuerung machen kann.

6. Vorrichtung nach Anspruch 3, die weiterhin Sensormittel umfasst, die den Steuermitteln anzeigen, wann das Schüttelelement in der ersten Position ist und wann das Schüttelelement in der zweiten Position ist, wobei die Sensormittel eine Codierscheibe umfassen, die mit dem Schüttelelement verdreht wird, wobei die Codierscheibe mindestens ein Merkmal aufweist, das von dem Reste der Codierscheibe unterscheidbar ist und wobei mindestens eine Sensoreinrichtung auf das mindestens eine Merkmal anspricht und ein Eingabesignal an die Steuermittel als Antwort auf die Ausrichtung des Merkmals auf die Sensoreinrichtung bereitstellt.

7. Vorrichtung nach Anspruch 6, wobei die Codierscheibe ein erstes Merkmal aufweist, das auf die Sensoreinrichtung ausgerichtet ist, wenn das Schüttelelement in der ersten Position ist, und ein zweites ähnliches Merkmal, das auf die Sensoreinrichtung ausgerichtet ist, wenn das Schüttelelement in der zweiten Position ist.

8. Vorrichtung nach Anspruch 3, die weiterhin Sensormittel umfasst, um den Steuermitteln anzuzeigen, wann der primäre Magnet in der ersten Position ist und wann der primäre Magnet in der zweiten Position ist.

9. Verfahren des Verwendens von paramagnetischen Mikroperlen, um eine magnetische Zelltrennung durchzuführen, das eine Folge einer Mehrzahl von Aktivitäten umfasst, wobei das Verfahren die Schritte aufweist:
Bereitstellen einer Maschine, die mit der Mehrzahl von Aktivitäten und ihrer Abfolge programmiert ist;
Verwenden der Maschine, um mehrere bestimmte der Mehrzahl von Aktivitäten aufeinanderfolgend auszuführen;
Verwenden der Maschine, um einen menschlichen Bediener aufzufordern, mehrere ausgewählte der Mehrzahl von Aktivitäten aufeinanderfolgend auszuführen; und
Fordern von dem menschlichen Bediener, eine Bestätigungseingabe an die Maschine nach der Vervollständigung jeder einzelnen aus der Gruppe der Mehrzahl von ausgewählten Aktivitäten zu machen;
Veranlassen, dass die Maschine eine der bestimmten Aktivitäten der Mehrzahl von Aktivitäten bei der magnetischen Zelltrennung auf das Empfangen einer entsprechenden Bestätigungseingabe von dem menschlichen Benutzer durch die Maschine, dass eine direkt vorangehende der ausgewählten Aktivitäten vervollständigt wurde, automatisch ausführt;
Verwenden der Maschine, um den menschlichen Bediener herbeizurufen, wenn die Maschine eine der bestimmten Aktivitäten in Vorbereitung dafür, dass der menschliche Bediener die nächste nachfolgende der mehreren ausgewählten Aktivitäten ausführt, abgeschlossen hat;
Verwenden der Maschine, um die bestimmten Schritte auszuführen: Schütteln eines primären Behälters, der eine Mischung von Zellen und paramagnetischen Mikroperlen in Flüssigkeit enthält; magnetisches Einfangen der paramagnetischen Mikroperlen in der Flüssigkeit, und magnetisches Loslassen der paramagnetischen Mikroperlen in der Flüssigkeit,
Versehen der Maschine mit einem Schüttelelement, das den primären Behälter trägt und das beweglich ist, um das Schütteln zu bewirken;
Bereitstellen eines primären Magnets an dem Schüttelelement, der zwischen einer ersten Position benachbart dem primären Behälter, um das magnetische Einfangen der paramagnetischen Mikroperlen zu bewirken, und einer zweiten Position beabstandet von dem primären Behälter, um das magnetische Freigeben der paramagnetischen Mikroperlen zu bewirken, beweglich ist, und
Bewegen des primären Magnets durch Kraftantriebsmittel zwischen der ersten und der zweiten Position unabhängig von dem Schütteln des primären Behälters durch Bewegung des Schüttelelements.

10. Verfahren nach Anspruch 9, das zusätzlich die Schritte umfasst:
Auslösen, dass die Maschine ein anfängliches Schütteln des primären Behälters ausführt, um ausgewählte Targetzellen aus der Mischung an die paramagnetischen Perlen zu binden, gefolgt von einem anfänglichen magnetischen Einfangen der paramagnetischen Perlen und der gebundenen Targetzellen in dem primären Behälter;
Verwenden der Maschine, um den Bediener aufzufordern, die Flüssigkeit aus dem primären Behälter in den Ablauf abzulassen und frische Flüssigkeit in den primären Behälter zuzugeben;
Auslösen, dass die Maschine die paramagnetischen Perlen magnetisch in die frische Flüssigkeit innerhalb des primären Behälters freigibt;
Auslösen, dass die Maschine den primären Behälter mit der frischen Flüssigkeit und den Targetzellen, die an die paramagnetischen Perlen gebunden sind, schüttelt, und dass sie die paramagnetischen Perlen und die gebundenen Targetzellen magnetische wieder einfängt;
Verwenden der Maschine, um einen Bediener aufzufordern, ein Material in den primären Behälter zu geben, das die Targetzellen von den paramagnetischen Perlen entbindet, Auslösen, dass die Maschine die paramagnetischen Perlen und die gebundenen Targetzellen in die frische Flüssigkeit und das dahinein zugegebene Material magnetisch freigibt, und Verwenden der Maschine, um den primären Behälter zu schütteln, um die Entbindung der Targetzellen von den paramagnetischen Perlen zu bewirken;
Auslösen, dass die Maschine die paramagnetischen Perlen in dem primären Behälter wieder einfängt, während die Targetzellen in der Flüssigkeit vermischt bleiben, und Verwenden der Maschine, um den Bediener aufzufordern, die Flüssigkeit und die Targetzellen aus dem primären Behälter in einem Produktsammelbehälter ablaufen zu lassen;
Einschließen eines sekundären Magnets in die Maschine;
Einschließen eines sekundären Behälters in die Vorrichtungsanordnung gegenüber dem sekundären Magnet;
Fließen lassen der Flüssigkeit und der Targetzellen zu dem Sammelbehälter durch den sekundären Behälter längs des sekundären Magnets, und
Verwenden des sekundären Magnets, um jegliche paramagnetische Mikroperlen, die aus dem primären Behälter entwichen sind, einzufangen.

11. Verfahren nach Anspruch 10, das zusätzlich die Schritte umfasst:
Verwenden der Maschine, um den Bediener aufzufordern, frische Flüssigkeit in den primären Behälter einzugeben, und
Verwenden der Maschine, um die paramagnetischen Perlen magnetisch zusammen mit jeglichen eingefangenen Targetzellen in die frische Flüssigkeit freizugeben, um ein Schütteln des primären Behälters zu bewirken, um jegliche eingefangenen Targetzellen von den paramagnetischen Perlen zu trennen, um die paramagnetischen Perlen magnetisch wieder einzufangen, während die Targetzellen in der Flüssigkeit vermischt bleiben, und Verwenden der Maschine, um den Bediener aufzufordern, die Flüssigkeit und die Targetzellen in den Produktbehälter abzuziehen.

12. Verfahren nach Anspruch 11, das zusätzlich den Schritt Verwenden der Maschine, um den Bediener aufzufordern, die Vorrichtungen zu schließen und die Vorrichtung von der Maschine zu entfernen, umfasst.

13. Verfahren nach Anspruch 9, das zusätzlich den Schritt Konfigurieren der Maschine, um den primären Behälter um eine Schwenkachse zu verkippen, um das Schütteln zu bewirken, umfasst.

14. Verfahren nach Anspruch 13, das weiterhin den Schritt Auslösen, dass die Maschine den primären Behälter aufeinanderfolgend in entgegengesetzten Richtungen bezüglich einer horizontalen Position des primären Behälters verschwenkt, um das Schütteln zu bewirken, umfasst.

15. Verfahren nach Anspruch 13, das auch die Schritte umfasst Bereitstellen der Maschine, um das Schüttelelement durch Betätigung der ersten Kraftantriebsmittel selektiv zu positionieren, und Auslösen, dass die Maschine den Schüttelbehälter in eine erste ausgewählte angewinkelte Position bezüglich sowohl der horizontalen Position als auch der vertikalen Position des Schüttelbehälters verschwenkt, um eine erste Phase des magnetischen Einfangens der paramagnetischen Perlen zu bewirken.

16. Verfahren nach Anspruch 15, wobei die erste ausgewählte angewinkelte Position für das magnetische Einfangen der paramagnetischen Perlen so gewählt wird, dass sie im wesentlichen 5° von der horizontalen Position weg ist.

17. Verfahren nach Anspruch 16, das weiterhin den Schritt Halten des primären Behälters in der ersten ausgewählten angewinkelten Position für einen ausgewählten Zeitraum nach der Bewegung des primären Magneten in seine erste Position , um die erste Phase des magnetischen Einfangens der paramagnetischen Perlen zu bewirken, aufweist.

18. Verfahren nach Anspruch 17, das auch den Schritt Auslösen, dass der erste Zeitraum im wesentlichen 30 Sekunden beträgt, umfasst.

19. Verfahren nach Anspruch 18, das zusätzlich den Schritt wahlweises Verschwenken des Schüttelelements in eine vertikale Position für den primären Behälter für eine zweite Phase des magnetischen Einfangens der paramagnetischen Perlen umfasst.

20. Verfahren nach Anspruch 19, das den Schritt Auslösen, dass die zweite Phase des magnetischen Einfangens der magnetischen Perlen eine Zeitdauer von im wesentlichen 2 min aufweist, umfasst.

21. Verfahren nach Anspruch 10, das zusätzlich den Schritt Verwenden der Maschine, um den Bediener aufzufordern, ein antikoagulierendes Material in die primäre Kammer vor dem anfänglichen Schütteln und dem Binden der Targetzellen an paramagnetischen Perlen hinzuzufügen, umfasst.

22. Verfahren nach Anspruch 10, das auch den Schritt Verwenden der Maschine, um dem Bediener anzuzeigen, nach dem anfänglichen Schüttelschritt und vor dem anfänglichen magnetischen Einfangen der paramagnetischen Perlen eine kleine Menge frischer Flüssigkeit in den primären Behälter zu geben, umfasst.

## Revendications

1. Appareil pour la séparation magnétique de cellules à l'aide de microbilles paramagnétiques, ledit appareil comprenant : une base, ladite base supportant de manière mobile un agitateur et un premier moyen de commande par moteur pour agiter ledit agitateur par rapport à ladite base ;
ledit agitateur incluant un moyen pour fixer de manière amovible un récipient primaire de séparation dans lequel une suspension liquide des cellules et des microbilles peut être agitée par le mouvement dudit agitateur ; ledit agitateur portant en outre de manière mobile un aimant primaire pour créer un mouvement entre une première position adjacente au récipient primaire fixé et dans lequel le flux magnétique venant dudit aimant primaire pénètre le récipient primaire pour attirer et capturer les microbilles et une seconde position écartée du récipient primaire de telle sorte que ledit flux magnétique ne capture pas les microbilles ; et un second moyen de commande par moteur pour déplacer de manière sélective ledit aimant primaire entre lesdites première et seconde positions indépendamment de l'agitation dudit récipient primaire ; et
incluant en outre ladite base portant un support pour un récipient secondaire en communication de fluide avec ledit récipient primaire et un aimant secondaire disposé de manière adjacente audit récipient secondaire pour capturer les microbilles paramagnétiques qui s'échappent dudit récipient primaire.

2. Appareil selon la revendication 1 dans lequel ladite base porte en pivotant ledit agitateur et est conformée de telle sorte qu'elle maintienne un récipient primaire allongé, ledit premier moyen de commande par moteur pouvant aussi incliner ledit agitateur et ledit récipient primaire entre une première position dans laquelle le récipient primaire est vertical et une seconde position dans laquelle le récipient primaire est horizontal et est agité par ledit premier moyen de commande par moteur appliquant audit agitateur et audit récipient primaire successivement des mouvements d'inclinaison opposés depuis ladite seconde position horizontale.

3. Appareil selon la revendication 2 dans lequel ledit premier moyen de commande par moteur est capable de faire bouger aussi ledit agitateur et ledit récipient- primaire dans une première position de séparation dans laquelle ledit agitateur et le récipient primaire sont placés dans une position formant un angle par rapport à ladite seconde position horizontale en fonction d'un certain angle.

4. Appareil selon la revendication 3 dans lequel ledit certain angle est sensiblement de 5 degrés.

5. Appareil selon la revendication 3 dans lequel ledit appareil inclut en outre un dispositif d'entrée d'utilisateur par lequel ledit utilisateur humain peut faire une entrée dans ledit contrôleur à microprocesseur.

6. Appareil selon la revendication 3 incluant en outre un moyen formant capteur indiquant audit moyen de contrôle le moment où ledit agitateur est dans ladite première position et le moment où ledit agitateur est dans ladite seconde position, ledit moyen formant capteur incluant un disque d'encodage tournant avec ledit agitateur, ledit disque d'encodage ayant au moins une caractéristique distincte par rapport au reste dudit disque d'encodage, et au moins un capteur répondant à ladite au moins une caractéristique et fournissant une entrée audit moyen de contrôle en réponse à l'alignement de ladite caractéristique avec ledit capteur.

7. Appareil selon la revendication 6 dans lequel ledit disque d'encodage dispose d'une première caractéristique qui s'aligne avec ledit capteur lorsque ledit agitateur est dans ladite première position et d'une seconde caractéristique similaire qui s'aligne avec ledit capteur lorsque ledit agitateur est dans ladite seconde position.

8. Appareil selon la revendication 3 incluant en outre un moyen formant capteur indiquant audit moyen de contrôle le moment où ledit aimant primaire est dans ladite première position et le moment où ledit aimant primaire est dans ladite seconde position.

9. Méthode d'utilisation de microbilles paramagnétiques pour procéder à une séparation magnétique de cellules qui inclut des activités multiples successives, ladite méthode comprenant les étapes suivantes :
fournir une machine programmée pour lesdites multiples activités et leur succession ;
utiliser ladite machine pour réaliser plusieurs desdites multiples activités successivement ;
utiliser ladite machine pour suggérer à un utilisateur humain de réaliser plusieurs desdites multiples activités sélectionnées successivement ; et
demander audit utilisateur humain de faire une entrée de vérification dans ladite machine après réalisation de chaque activité individuelle ou d'un groupe desdites plusieurs activités choisies ;
faire réaliser automatiquement à ladite machine une desdites certaines activités desdites activités multiples de ladite séparation magnétique de cellules à réception par ladite machine d'une entrée respective de vérification par ledit utilisateur humain signalant qu'une desdites activités choisies immédiatement précédente a été réalisée ;
utiliser ladite machine pour signaler audit utilisateur humain le moment où ladite machine a terminé l'une desdites certaines activités préparant la réalisation par ledit utilisateur humain de l'activité suivante desdites plusieurs activités choisies ;
utiliser ladite machine pour réaliser les certaines étapes suivantes : agitation d'un récipient primaire contenant un mélange de cellules et de microbilles paramagnétiques dans un liquide ; capture magnétique desdites microbilles paramagnétiques dans ledit liquide ; et libération magnétique desdites microbilles paramagnétiques dans ledit liquide ;
équipement de ladite machine avec un agitateur qui porte ledit récipient primaire et qui peut être déplacé pour effectuer ladite agitation ;
équipement dudit agitateur avec un aimant primaire qui peut être déplacé entre une première position adjacente audit récipient primaire pour effectuer ladite capture magnétique desdites microbilles paramagnétiques et une seconde position écartée dudit récipient primaire pour effectuer la libération magnétique desdites microbilles paramagnétiques ; et
déplacement dudit aimant primaire par un moyen de commande par moteur entre lesdites première et seconde positions indépendamment de l'agitation dudit récipient primaire par mouvement dudit agitateur.

10. Méthode selon la revendication 9 incluant en outre les étapes suivantes : faire que la machine réalise une agitation initiale dudit récipient primaire pour fixer des cellules cibles choisies dans ledit mélange auxdites microbilles paramagnétiques, puis capture magnétique initiale desdites microbilles paramagnétiques et des cellules cibles fixées dans ledit récipient primaire ;
utiliser ladite machine pour suggérer audit utilisateur de drainer ledit liquide en dehors dudit récipient primaire pour élimination et d'ajouter du liquide frais dans ledit récipient primaire ;
faire que ladite machine libère magnétiquement lesdites microbilles paramagnétiques dans ledit liquide frais dans ledit récipient primaire ;
ensuite faire que ladite machine agite ledit récipient primaire avec ledit liquide frais et les cellules cibles fixées auxdites microbilles paramagnétiques et qu'elle recapture magnétiquement lesdites microbilles paramagnétiques et les cellules cibles fixées ;
utiliser ladite machine pour suggérer audit utilisateur d'ajouter un matériau audit récipient primaire qui défera la fixation des cellules cibles auxdites microbilles paramagnétiques, entraînant la libération magnétique par ladite machine desdites microbilles paramagnétiques et cellules cibles fixées dans ledit liquide frais et dans le matériau ajouté, et utiliser ladite machine pour agiter ledit récipient primaire pour défaire la fixation desdites cellules cibles desdites microbilles paramagnétiques ;
faire que ladite machine recapture lesdites microbilles paramagnétiques dans ledit récipient primaire pendant que lesdites cellules cibles restent mélangées dans ledit liquide et utiliser ladite machine pour suggérer audit utilisateur de drainer ledit liquide et lesdites cellules cibles dudit récipient primaire vers un récipient de collecte de produit ;
inclure un aimant secondaire sur ladite machine ; inclure dans ledit appareil un récipient secondaire juxtaposé audit aimant secondaire ;
faire couler ledit liquide et lesdites cellules cibles dans ledit récipient de collecte à travers ledit récipient secondaire en passant devant ledit aimant secondaire ; et
utiliser ledit aimant secondaire pour capturer toute microbille paramagnétique s'étant échappée dudit récipient primaire.

11. Méthode selon la revendication 10 incluant en outre les étapes suivantes : utiliser ladite machine pour suggérer audit utilisateur d'ajouter du liquide frais dans ledit récipient primaire, et utiliser ladite machine pour libérer magnétiquement lesdites microbilles paramagnétiques avec les cellules cibles piégées dans ledit liquide frais, de lancer l'agitation dudit récipient primaire pour séparer toute cellule cible piégée desdites microbilles paramagnétiques, de recapturer magnétiquement lesdites microbilles paramagnétiques tandis que lesdites cellules cibles restent mélangées dans ledit liquide et utiliser ladite machine pour suggérer audit utilisateur de drainer ledit liquide et lesdites cellules cibles dans ledit récipient de collecte.

12. Méthode selon la revendication 11 incluant en outre les étapes suivantes : utiliser la machine pour suggérer audit utilisateur de fermer ledit appareil et de retirer ledit appareil de ladite machine.

13. Méthode selon la revendication 9 incluant en outre les étapes suivantes : configurer ladite machine pour incliner ledit récipient primaire autour d'un axe pivot pour entraîner ladite agitation.

14. Méthode selon la revendication 13 incluant en outre les étapes suivantes : faire que ladite machine incline ledit récipient primaire successivement dans des directions opposées par rapport à une position horizontale dudit récipient primaire afin d'entraîner ladite agitation.

15. Méthode selon la revendication 13 incluant aussi les étapes de faire que ladite machine place de manière sélective ledit agitateur en faisant fonctionner ledit premier moyen de commande par moteur et de faire que ladite machine incline ledit récipient d'agitation dans une première position choisie formant un angle par rapport à la position horizontale et à une position verticale dudit récipient d'agitation pour effectuer une première phase de ladite capture magnétique desdites microbilles paramagnétiques.

16. Méthode selon la revendication 15 dans laquelle ladite première position choisie formant un angle pour ladite capture magnétique desdites microbilles paramagnétiques est choisie pour former un angle sensiblement de 5 degrés par rapport à ladite position horizontale.

17. Méthode selon la revendication 16 incluant en outre l'étape suivante : maintenir ledit récipient primaire dans ladite première position choisie formant un angle pendant une durée choisie suivant le déplacement dudit aimant primaire dans ladite première position pour effectuer ladite première phase de capture magnétique desdites microbilles paramagnétiques.

18. Méthode selon la revendication 17 incluant l'étape suivante : faire en sorte que la première durée soit sensiblement de 30 secondes.

19. Méthode selon la revendication 18 incluant en outre l'étape suivante : incliner de manière sélective ledit agitateur en une position verticale pour ledit récipient primaire pour une seconde phase de ladite capture magnétique desdites microbilles paramagnétiques.

20. Méthode selon la revendication 19 incluant l'étape suivante : faire en sorte que la seconde phase de ladite capture magnétique desdites microbilles magnétiques dure sensiblement 2 minutes.

21. Méthode selon la revendication 10 incluant en outre l'étape suivante : utiliser ladite machine pour suggérer audit utilisateur d'ajouter un anticoagulant dans ladite chambre primaire avant ladite agitation initiale et de fixer lesdites cellules cibles auxdites microbilles paramagnétiques.

22. Méthode selon la revendication 10 incluant aussi l'étape suivante : utiliser ladite machine pour suggérer audit utilisateur d'ajouter une petite quantité de liquide frais dans ledit récipient primaire après ladite étape d'agitation initiale et avant ladite capture magnétique initiale desdites microbilles paramagnétiques.
